(19) Europäisches Patentamt
European Patent Office
Office européen des brevets

(11) EP 1 961 764 B1

(12) **EUROPEAN PATENT SPECIFICATION**

(45) Date of publication and mention of the grant of the patent:
**11.05.2011 Bulletin 2011/19**

(21) Application number: **06833637.9**

(22) Date of filing: **29.11.2006**

(51) Int Cl.:
***C07K 14/605*** (2006.01) ***A61K 38/00*** (2006.01)
***A61P 3/10*** (2006.01) ***C07K 1/04*** (2006.01)
***C08B 37/00*** (2006.01)

(86) International application number:
**PCT/JP2006/323834**

(87) International publication number:
**WO 2007/063907 (07.06.2007 Gazette 2007/23)**

(54) **SUGAR CHAIN ADDUCT OF PEPTIDE AND PHARMACEUTICAL COMPRISING THE SAME AS ACTIVE INGREDIENT**

ZUCKERKETTENADDUKT EINES PEPTIDS UND PHARMAZEUTIKUM, DAS DIESES ALS WIRKSTOFF ENTHÄLT

PRODUIT D'ADDITION DE CHAINE DE SUCRES ET DE PEPTIDE ET PRODUIT PHARMACEUTIQUE COMPRENANT CE PRODUIT EN TANT QUE PRINCIPE ACTIF

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.11.2005 JP 2005346905**

(43) Date of publication of application:
**27.08.2008 Bulletin 2008/35**

(73) Proprietors:
- **Shionogi Co., Ltd.**
  **Osaka-shi, Osaka 541-0045 (JP)**
- **National University Corporation Hokkaido University**
  **Sapporo-shi**
  **Hokkaido 060-0808 (JP)**

(72) Inventors:
- **ITO, Takaomi,**
  **c/o Shionogi Innovation Center for Drug Discovery**
  **Sapporo-shi, Hokkaido 001-0021 (JP)**
- **TAKIMOTO, Akio**
  **Amagasaki-shi, Hyogo 660-0813 (JP)**
- **NAGATOME, Hirofumi**
  **Osaka-shi, Osaka 553-0002 (JP)**
- **FUMOTO, Masataka**
  **Osaka-shi, Osaka 553-0002 (JP)**
- **UEDA, Taichi**
  **Osaka-shi, Osaka 553-0002 (JP)**
- **NISHIMURA, Shin-Ichiro**
  **Sapporo-shi, Hokkaido 0010021 (JP)**

(74) Representative: **Hayes, Adrian Chetwynd**
**Boult Wade Tennant**
**Verulam Gardens**
**70 Gray's Inn Road**
**London WC1X 8BT (GB)**

(56) References cited:
**WO-A-2006/010143** **WO-A1-91/11457**
**WO-A1-2004/037859** **WO-A2-02/46227**

- **J A MEURER ET AL.: "Properties of native and in vitro glycosylated forms of the glucagon-like peptide-1 receptor antagonist exendin (9-39)" METABOLISM, CLINICAL AND EXPERIMENTAL., vol. 48, no. 6, June 1999 (1999-06), pages 716-724, XP009019813 US W.B. SAUNDERS CO., PHILADELPHIA, PA.**
- **BURCELIN R. ET AL.: 'Long-lasting antidiabetic effect of adipeptidyl peptidase IV-resistant analog of glucagon-likepeptide-1' METABOLISM vol. 48, no. 2, 1999, pages 252 - 258, XP003013281**
- **RITZEL U. ET AL.: 'A synthetic glucagon-like peptide-1 analog with improved plasma stability' JOURNAL OF ENDOCRINOLOGY vol. 159, 1998, pages 93 - 102, XP003013282**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MARK M.H. ET AL.: 'Evaluation of glycated glucagon- like peptide-1(7-36) amide in intestinal tissue of normal and diabetic animal models' BIOCHIMICA ET BIOPHYSICA ACTA vol. 1569, 2002, pages 75 - 80, XP003013283
- O'HARTE F.P.M. ET AL.: 'Degradation and glycemic effects of His7-glucitol glucagon-like peptide-1(7-36) amide in obeseob/ob mice' REGULATORY PEPTIDES vol. 96, 2001, pages 95 - 104, XP003013284
- O'HARTE F.P.M. ET AL.: 'N-terminally modified glucagon-like peptide-1(7-36) amide exhibits resistance to enzymatic degradation while maintaining its anti hyperglycaemic activity in vivo' BIOCHIMICA ET BIOPHYSICA ACTA vol. 1474, 2000, pages 13 - 22, XP003013285
- O'HARTE F.P.M. ET AL.: 'Amino terminal glycation of gastric inhibitory polypeptide enhances its insulintropic action on clonal pancreatic B-cells' BIOCHIMICA ET BIOPHYSICA ACTA vol. 1425, 1998, pages 319 - 327, XP003013286
- O'HARTE F.P.M. ET AL.: 'NH2-Terminally Modified Gastric Inhibitory Polypeptide Exhibits Amino-Peptidase Resistance and Enhanced Antihyperglycemic Activity' DIABETES vol. 48, 1999, pages 758 - 765, XP003013287
- O'HARTE F.P.M. ET AL.: 'Glycated Cholecystokinin-8 Hasan Enhanced Satiating Activity and Its Protected Against Enzymatic Degradation' DIABETES vol. 47, 1998, pages 1619 - 1624, XP003013288
- ORSKOV C. ET AL.: 'Complete Sequences of Glucagon-like Peptide-1 from Human and Pig Small Intestine' J. BIOL. CHEM. vol. 264, no. 22, 1989, pages 12826 - 12829, XP003013289
- THOMA R. ET AL.: 'Structural Basis of Proline-Specific Exopeptidase Activity as Observed in Human Dipeptidyl Peptidase-IV' STRUCTURE vol. 11, 2003, pages 947 - 959, XP003013290
- O'HARTE F.P.M. ET AL.: 'Glycation of glucagon-like peptide-1 (7-36) amide: characterization and impared action on rat insulin secreting cells' DIABETOLOGIA vol. 41, 1998, pages 1187 - 1193, XP003013291
- O'HARTE F.P.M. ET AL.: 'Effects of non-glycated and glycated glucagon-like peptide-1 (7-36) amide on glucose metabolism in isolated mouse abdominal muscle' PEPTIDES vol. 18, no. 9, 1997, pages 1327 - 1333, XP003013292

## Description

TECHNICAL FIELD

This invention relates to a novel glycosylated peptide and a medicine comprising it as an effetive ingredient. In detail, it relates to a novel glycosylated peptide relating to a glucagon-like peptide-1(GLP-1), which stimulates insulin secretion and is useful as a medicine for treating diabetes.

BACKGROUND ART

Glucagon-like peptide-1(GLP-1) is a peptide hormone secreted from L-cells in the small intestine into blood composed of 30 amino acid residues(Non-patent literature 1). GLP-1 is expected as a candidate of medicine treating diabetes since it stimulates insulin secretion in glucose concentration-dependent manner and has an activity to suppress glucagon secretion, appetite and excretion of gastric emptying(Non-patent literature 2). However, native GLP-1 is degraded in vivo by dipeptidyl peptidase IV(DPP-IV), which releases the N-terminal dipeptide, His-Ala, and inactivates GLP-1(Non-patent literature 3 and 4), and the half-life of GLP-1 in blood is only several minutes(Non-patent literature 5). Therefore its clinical application was so difficult.
In the past, many GLP-1 derivatives have been reported, which acquired resistance to DPP-IV is aquired by substitution and/or modification of amino acid residues around the cleaved site by DPP-IV. For example, the GLP-1 derivatives which include modification of $His^7$ of the N-terminus (Non-patent literature 6-8), amino acid substitution of $Ala^8$(Non-patent literature 9-11) or Glu (Non-patent literature 12) have been reported.
Non-patent literature 1: Lancet. 19872 1300-4
Non-patent literature 2: Regul Pept 2005; 128: 135-48
Non-patent literature 3: Eur J Biochem 1993; 214: 829-35
Non-patent literature 4: J Biol Chem 1997; 272: 21201-6
Non-patent literature 5: Diabetologia 1998; 41: 271-278
Non-patent literature 6: Regul Pept 2001; 96: 95-104
Non-patent literature 7: Regul Pept 2000; 86: 103-111
Non-patent literature 8: Regul Pept 1999; 79:93-102
Non-patent literature 9: JBC 2004; 279: 3998-4006
Non-patent literature 10: J Endocrinol 1998; 159: 93-102
Non-patent literature 11: Metabolism 1999; 48:252-258
Non-patent literature 12: Biol Chem 2003; 384: 1543-1551

DISCLOSURE OF INVENTION

PROBLEM TO BE SOLVED

The objective of the present invention is to provide a derivaive of GLP-1 related peptide, which has a long half-life in blood and is useful as a stimulator of insulin secretion.

MEANS TO SOLVE THE PROBLEM

The inventors have found that it is successful to provide a DPP-IV tolerant GLP-1 derivative while maintaining an activity to stimulate insulin secretion by glycosylation of GLP-1 related peptides, and completed the present invention.

EFFECT OF INVENTION

The glycosylated GLP-1 related peptides have a long half-live in blood and continuously stimulate insulin secretion.

BRIEF DESCRIPTION OF DRAWINGS

In the figure, the result of a MS spectroscopy was shown as to the prepared glycosylated peptides.

Figure 1: The result of MS spectroscopy as to glycosylated GL34N was shown.

BEST MODE FOR CARRYING OUT THE INVENTION

A glycosylated peptide described herein is a glycopeptide in which glycochains set forth below are attached to GLP-1 related peptides. The term of "GLP-1 related peptide" means GLP-1 (7-36) amide of the formula(I) or Excendin-4 derivative as claimed in claims 1-2

GLP-1 related peptide has an activity to stimulate insulin secretion and His$^{7}$, Gly$^{10}$, Phe$^{12}$, Thy$^{13}$, Asp$^{15}$, Phe$^{28}$ and Ile$^{29}$ in the peptide(I), and His$^{1}$, Gly$^{4}$, Phe$^{6}$, Thr$^{7}$, Asp$^{9}$, Phe and Ile$^{23}$ in the peptide(II) are important for expressing the activity.

Next, embodiment of glycosylation is set forth. Glycochain may be attached directly or through a liker to an functional group of amino acid side chain. Specifically as shown in the next formula,

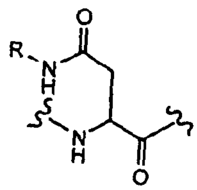

(c)

,

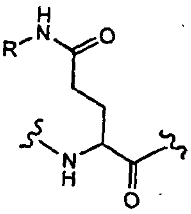

(d)

,

,

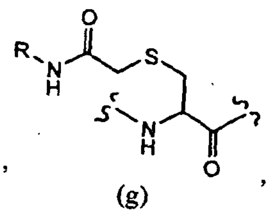

(g)

,

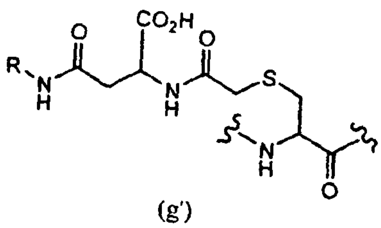

(g')

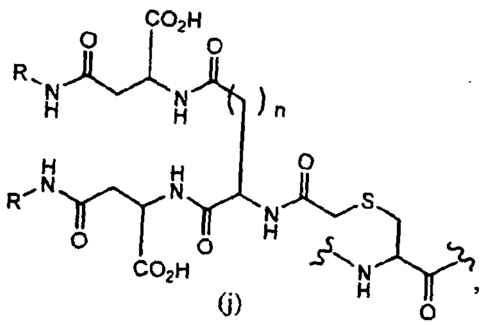

(j)

,

wherein n, is an integer of 1 to 10,

glycosylation is possible at the side chain of Asp, Asn, Glu, Gln, Ser, Thr and/or Cys. The term "glycosylated Ser, Thr, Asp, Asn, Glu, Gln and Cys" include groups shown by the formulae above.

It is known that the penultimate amino acid residue at the N-terminus in the natural GLP-1(7-36)amide is enzymatically cleaved by DDP-IV. Accordingly, it is preferable that the glycosylation site is as close to the cleaved site as possible unless it affect the activity.

Examples of the glycochain used in the present invention are set forth below; Examples of the

GlcNAc

Galβ1-4GlcNAc

NeuAcα2-6Galβ1-4GlcNAc

Galβ1-4(Fucα1-3)GlcNAc

NeuAca2-3Galpl-4GlcNAc

NeuAcα2-3Galβ1-4(Fucα1-3)GlcNAc

NeuAcm2-6Galpl-4(Fucal-3)GlcNAc

GlcNAcβ1(-3Galβ1-4GlcNAcβ1)$_n$-3Galβ1-4GlcNAc
Galβ1-4GlcNAcβ1(-3Galβ1-4GlcNAcβ1)$_n$-3Galβ1-4GlcNAc
NeuAcα2-3Galβ1-4GlcNAcβ1(-3Galβ1-4GlcNAcβ1)$_n$-3Galβ1-4GlcNAc
NeuAcα2-6Galβ1-4GlcNAcβ1(-3Galβ1-4GlcNAcβ1)$_n$-3Galβ1-4GlcNAc

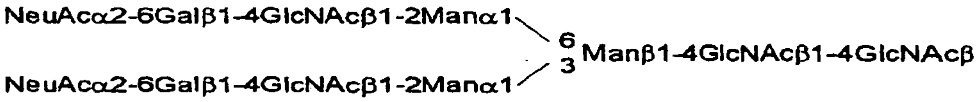

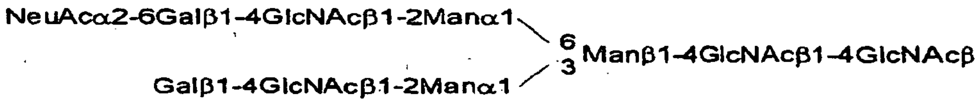

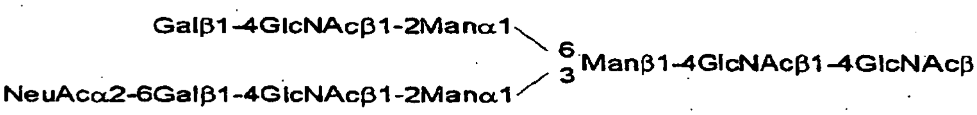

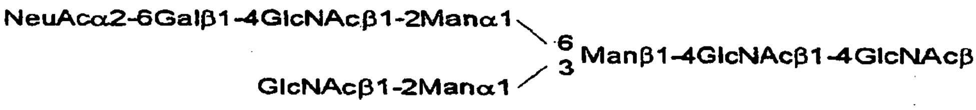

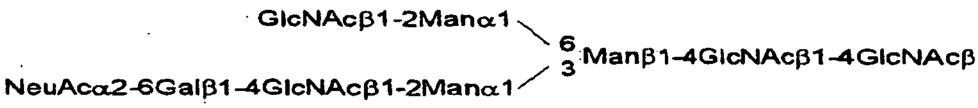

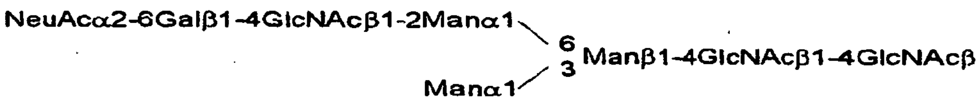

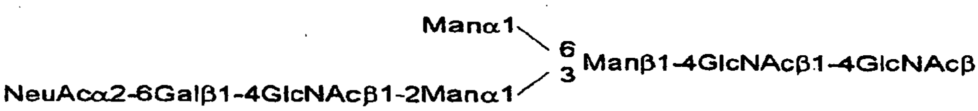

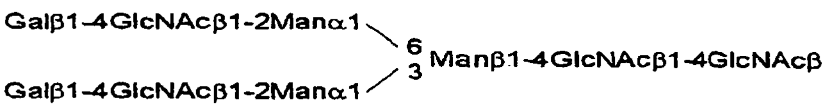

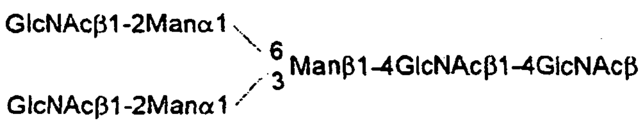

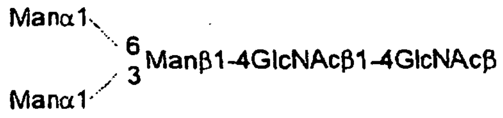

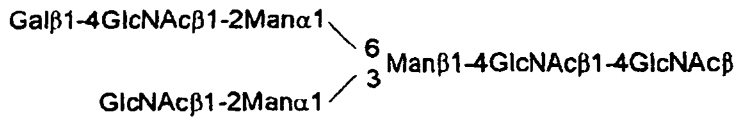

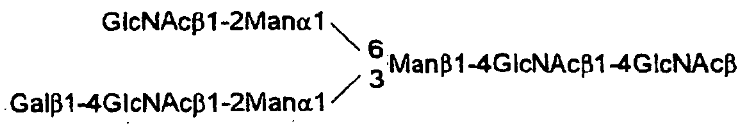

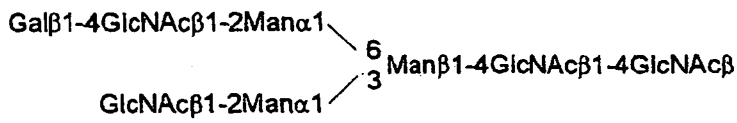

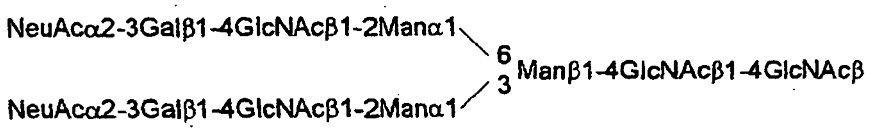

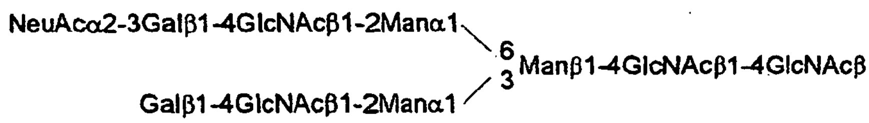

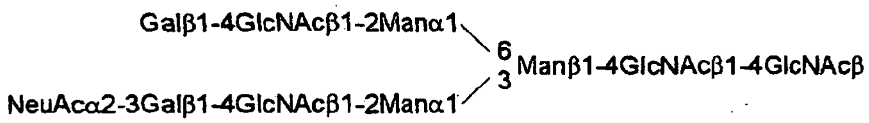

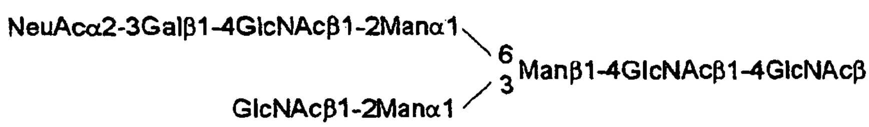

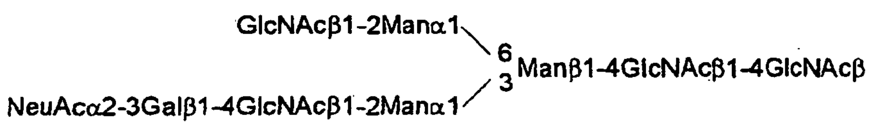

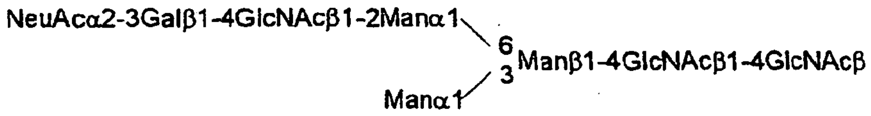

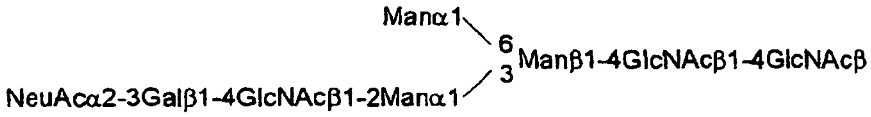

NeuAcα2-6(NeuAca2-3)Galβ1-4GlcNAc, or Gen.

Examples of especially preferred glycosylated peptide include a derivative in which the especially preferred glycochain is attached to the preferred peptide of the formula(I), (II) and (III) described above..

In the specification, the term "degradative enzyme" means an enzyme involved in metabolism of GLP-1 related peptide such as DPP-IV, neutral endopeptidase and the like.

Peptide chain of GLP-1 related peptide can be appropriately synthesized by a solid phase peptide synthesis using Boc-method or Fmoc-method. Glycosylation may be carried out by the silid phase peptide synthesis usung a monosaccharide of aminoacid such as Asn(GlcNAc) and subsequent additional modification of the glycochain, if necessary. A glycochain may be elongated by glycoltransferase etc.

Specifically, an Asn residue of the said peptide chain, the side chain of which is glycosylated(Asn-type), may be synthesized as followed;

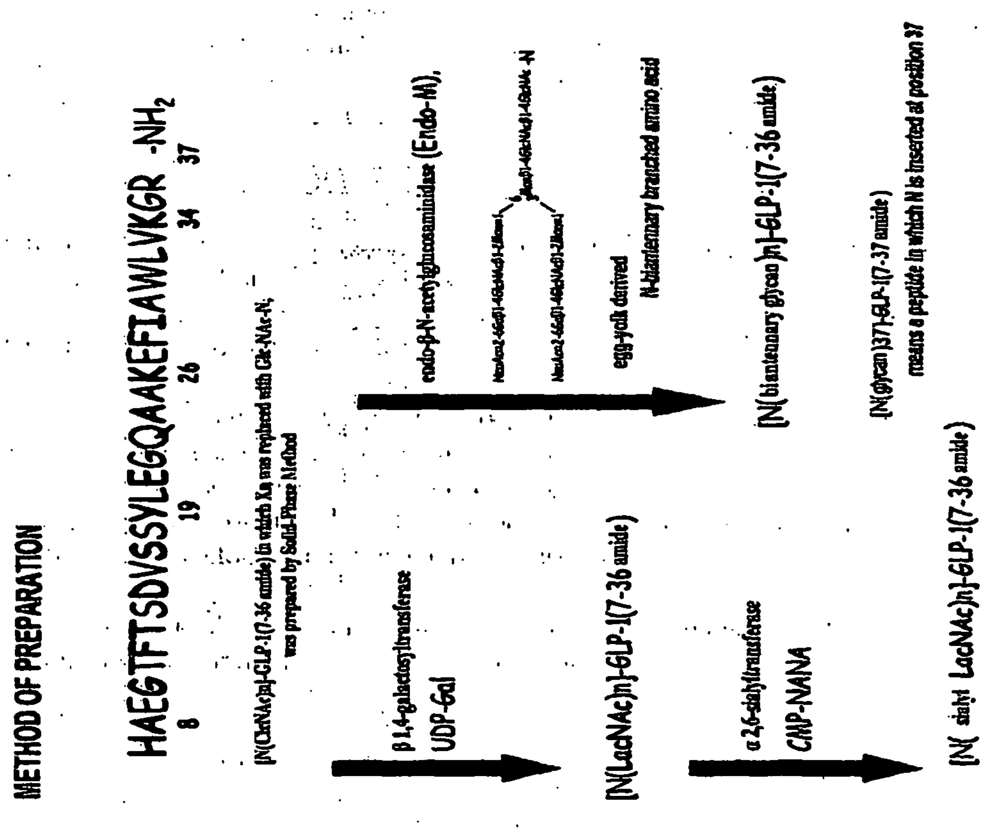


Also, a glycosylated derivative in which the side chain of Cys residue is glycosylated(Cys-type) may be synthesized by a general scheme:

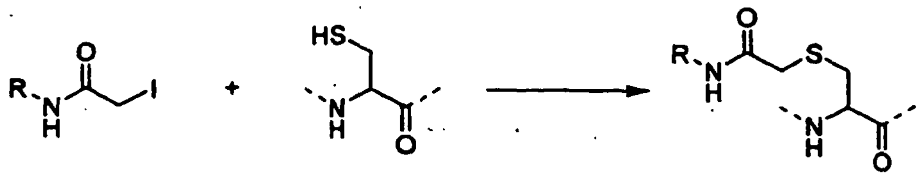

wherein the Cys-substituted peptide prepared by the solid phase peptide symthesis is coupled with the iodoacetyl derivative prepared by a chemical synthesis. In the scheme, the dotted line means the peptide chain of the GLP-1 related peptide and R is a glycochain.

The derivative containing the bitantennary-N-glycan is obtained by a method using endo-M enzyme or the reaction of the compound(3) of Reference Example and Cys-substituted peptide.

The derivative containing the biantennary-N-glycan dimer is obtained by the reaction of the compound(7) of Reference Example and Cys-substituted peptide.

The derivative containing the galactose trimer is obtained by the reaction of the compound(21) of Reference Example and Cys-substituted peptide.

Test Examples

Activities of the glycosylated peptide of the present invention to stimulate insulin secretion were evaluated by an agonistic activity (production of cAMP) and a receptor binding assay. Additionally, a prolonged activity of each glycosylated compound was evaluated by measuring a kinetic parameter of enzyme degradation caused by DPP-IV with GLP-1 derivatives, or testing a hypoglycemic activity with exendin 4 derivatives, which are resistant to DPP-IV.

1. Assay for producion of cAMP

CHO cells in which GLP-1 receptor was forced to express were seeded into 384-well plate in a concentration of 4000 cells/well and incubated for 48 hours. After being washed with the assay buffer(Hanks/20mM HEPES, pH 7.4, 0.1%BSA) three times, the assay buffer(20 μL) was added to the cells and further 10μL of a solution of GLP-1 derivative prepared with the assay buffer containing 0.1 mM IBMX and 0.2 mM R020-1724(final concentration: $10^{-12}$-$10^{-6}$M) was added. After stirring at room temperature for an hour, the cells were lysed with Triton X-100(final concentration: 1 %).

Quantity of cAMP was determined using cAMP Femtomolar Kit(CIS Bio International). The reaction solution (1μL) was moved to a new 384-well plate and diluted by adding 9μL of dilution buffer. Next, each 5 μL of cAMP-XL665 solution and anti-cAMP cryptate solution of the Kit was added, the mixture was incubated at room temperature for an hour, and time-resolved fluorescence was measured using RUBY star (BMG LABTECH). The amount of formed cAMP was calculated based on the calibration curve of cAMP. A 100% activity was assigned to the maximam amount of cAMP produced by GLP-1, and a concentration to give a 50% activity was adopted as an $ED_{50}$ value of the tested compound.

2. Receptor binding Assay

A membrane fraction(5 μL) prepared in the usual manner from CHO cells in which expression of GLP-1 receptor is forced was incubated with 62 pM[$^{125}$I]GLP-1(7-36) (Perkin-Elmer), 25mM HEPES, 5 mM MgCl, 1 mM $CaCl_2$, 0.25 mg/mL bacitracin, 0.1 % BSA, and GLP-1 derivative(final conc. $10^{-11}$ to $10^{-6}$M)(pH 7.4). After being incubated at room temperature for 2 hours, the solution was filtered through a unifilter 96GF/C plate(Perkin-Elmer) pretreated with 1% polyethylenimine containing 0.5 % BSA, washed with 25 mM HEPES buffer solution(pH 7.4) containing 0.5 % BSA, and radioactivity remained on the filter was measured by a gamma counter(Top Counter, Perkin-Elmer). An amount of binding under the presence of GLP-1(1 μL) was considered as a non-specific binding. A concentration to give a 50% displacement of the specific binding of [$^{125}$I]GLP-1(7-36) was adopted as an $IC_{50}$ value of each GLP-1 derivative.

3. Assay for Degradation Kinetics

GLP-1 analogue(20-500 mM) was incubated at 37°C with 0.7 μg/mL recombinant human DPP-IV in 100 mMHEPES buffer containing 0.05% Tween 80 and 1 mM EDTA·2Na(pH 7.5)(60 μL). The reaction was carried out in a polypropylene tube having a volume of I mL immersed in a temperature controlled bath at 37°C. During the first 25 minutes, 7.0 μL of the reaction solution was sampled in every 5 minute ,and concentration of a degradation product, a fragment peptide of the C terminus of the GLP-1 derivative produced by DPP-TV, was determined using HPLC. Develosil RPAQUEOUS-AR-3 (2.0×100 mm, Nomura Kagaku) was used as a column and the concentration was calculated besed on the UV absorvance at 210 nm. The initial velocity of the degradation reaction was determined from a slope of the

linear part obtained by plotting product concentration versus time. The initial velocity and concentration of the GLP-1 derivative were applied to Michaelis-Menten equation (1) and kinetics parameters, $k_{cat}$ and $K_M$ were determined as to each GLP-1 derivative.

$$V = \frac{k_{cat} \cdot E \cdot S}{K_M + S} \qquad (1)$$

E: Enzyme concentration [M],
$k_{cat}$: Reaction rat constant [$s^{-1}$],
$K_M$: Michaelis Constant [M],
S: Substrate concentration [M],
V: Initial Velocity [$Ms^{-1}$]

4. Assay for Hypoglycemic Activity

Natural excendin-4 or its glycosylated derivative was administered (1 or 100 nmol/kg, s.c.) to a male BKS.Cg-+Leprdb/+Leprdb mouse of 12-17 weeks (CLEA Japan, Inc.) and blood glucose level was monitored using Glucocard (Arkray) after the administration. In a control group, only a solvent was administered. Animals were fasted from 1.5 hours before to the end of the experiments in a group of 1 nmol/kg administration while a group of 100 nmol/kg administration was under ad libitium fed condition, and blood samples were taken from tail vein.
Blood glucose level of each time was compared to that of a control group and it was judged "significant" if risk rate P is <0.05 by Tukey Test, and evaluation "A" means a compound wherein sustained period of the significant hypoglycemic activity is longer than that of the same dose of excendin-4, and evaluation "B" means a compound wherein it is the same as excendin-4. As for EX(1-28)NG and EX(1-28)NS6, the activity was compared to EX(1-28).
Results of cAMP-production assay and receptor-binding assay, and kinetic parameters useful for evaluating resistance against enzymatic degradation are shown in Table 1.

[Tab 1]

| compounds | In vitro activity | | resistance 1) | |
|---|---|---|---|---|
| | $EC_{50}$ 2) [nM] | $IC_{50}$ 3) [nM] | $K_M$ [μM] | $k_{cat}/K_M$ [$10^4$/Ms] |
| GL | 0.11 | 0.41 | 27 | 14 |
| GL08N | 12 | 50 | 860 | 0.06 |
| GL08NG | > 1000 | > 100 | ND | ND |
| GL08NL | 284 | > 100 | ND | ND |
| GL08NS6 | 583 | > 100 | ND | ND |
| GL08NE1 | 200 | 420 | ND | ND |
| GL19N | 0.92 | 11 | 65 | 9.1 |
| GL19NG | 8.10 | 41 | 110 | 4.1 |
| GL19NL | 8.30 | 38 | 200 | 2.8 |
| GL19NS6 | 50 | > 100 | 970 | 1.1 |
| GL19NE1 | 75 | 850 | 2500 | 0.23 |
| GL26N | 0.09 | 1.40 | 110 | 4.9 |
| GL26NG | 0.28 | 2.80 | 180 | 2.5 |
| GL26NL | 0.33 | 2.00 | 230 | 2.0 |
| GL26NS6 | 0.92 | 2.90 | 1000 | 0.92 |
| GL26NE1 | 0.80 | 11 | 2000 | 0.25 |
| GL34N | 0.11 | 1.00 | 91 | 4.9 |
| GL34NG | 0.17 | 0.85 | 160 | 2.1 |
| GL34NL | 0.11 | 0.76 | 150 | 2.5 |

(continued)

| compounds | In vitro activity | | resistance 1) | |
|---|---|---|---|---|
| | $EC_{50}$ 2) [nM] | $IC_{50}$ 3) [nM] | $K_M$ [μM] | $k_{cat}/K_M$ [$10^4$/Ms] |
| GL34NS6 | 0.16 | 0.70 | 760 | 1.2 |
| GL34NE1 | 0.16 | 2.70 | 520 | 0.44 |
| GL34CE1 | 0.72 | 3.5 | - | - |
| GL34CE2 | 3.40 | 20 | - | - |
| GL37N | 0.09 | 0.72 | 18 | 13 |
| GL37NG | 0.11 | 0.53 | 71 | 7.7 |
| GL37NL | 0.12 | 0.47 | 75 | 6.3 |
| GL37NS6 | 0.11 | 0.71 | 290 | 2.1 |
| GL37NS3 | 0.21 | 0.45 | - | - |
| GL37NS36 | 0.35 | 0.82 | - | - |
| GL37NE1 | 0.18 | 2.50 | 330 | 1.0 |
| GLSGSGS43NS6 | 0.11 | 1.00 | - | - |
| GL2634NS6 | 2.2 | 27 | - | - |
| GL2637NS6 | 1.0 | 21 | - | - |
| GL3437NG | 0.11 | 0.90 | 210 | 2.5 |
| GL3437NL | 0.11 | 0.92 | 250 | 1.8 |
| GL3437NS6 | 0.20 | 3.0 | 1400 | 0.21 |
| GL3437NS3 | 0.06 | 2.6 | - | - |
| GL3437NS36 | 0.45 | 4.1 | - | - |
| GL3437CE1 | 4.1 | 30 | >1000 | <0.10 |
| GL263437NS6 | 6.1 | 51 | - | - |
| **1) kinetic parameters of GLP-1 degradation by DPPIV: ND means no degradation product<br>2) cAMP assay 3) receptor-binding assay** | | | | |

Results of cAMP-production assay and receptor-binding assay, and assay for sustained hypoglycemic *in vivo* activity are shown in Table 2-1 and 2-2.

[Tab 2-1]

| compound | In vitro activity [nM] | | In vivo activity duration |
|---|---|---|---|
| | $EC_{50}$ | $IC_{50}$ | |
| EX | 0.55 | 0.1 | B |
| EX-01 NG | > 10 | 23 | - |
| EX01NG | > 10 | 27 | - |
| EX02NG | > 10 | > 100 | - |
| EX03NG | > 100 | 39 | - |
| EX04NG | > 10 | 25 | - |
| EX05NG | > 100 | 27 | - |
| EX06NG | > 10 | > 100 | - |
| EX07NG | > 10 | 78 | - |
| EX08NG | > 100 | 35 | - |
| EX09NG | > 10 | 68 | - |
| EX10NG | > 10 | 36 | - |
| EX11 NG | > 10 | 28 | - |
| EX12NG | 0.26 | 1.2 | - |
| EX13NG | 0.53 | 2.3 | - |

(continued)

| compound | In vitro activity [nM] | | In vivo activity duration |
|---|---|---|---|
| | $EC_{50}$ | $IC_{50}$ | |
| EX14NG | > 10 | 14 | - |
| EX15NG | > 10 | > 100 | - |
| EX16NG | 0.22 | 1.0 | - |
| EX17NG | 0.084 | 0.38 | - |
| EX18NG | > 10 | 76 | - |
| EX19NG | > 10 | > 100 | - |
| EX20NG | 0.31 | 1.2 | - |
| EX21NG | 0.094 | 0.35 | - |
| EX22NG | > 10 | > 100 | - |
| EX23NG | > 10 | > 100 | - |
| EX24NG | 0.10 | 0.35 | - |
| EX25NG | 0.68 | 2.9 | - |
| EX26NG | > 10 | > 100 | - |
| EX27NG | 6.4 | 21 | - |
| EX28NG | 0.071 | 0.64 | B |
| EX29NG | 0.23 | 0.97 | - |
| EX30NG | 0.12 | 0.87 | - |
| EX31 NG | 0.11 | 0.45 | - |
| EX32NG | 0.13 | 0.49 | - |
| EX33NG | 0.079 | 0.50 | - |
| EX34NG | 0.066 | 0.42 | - |
| EX35NG | 0:10 | 0.37 | - |
| EX36NG | 0.083 | 0.34 | - |
| EX37NG | 0.14 | 0.46 | - |
| EX38NG | 0.089 | 0.42 | - |
| EX39NG | 0.077 | 0.48 | - |
| EX40NG | 0.079 | 0.46 | - |
| EX12NS6 | 0.84 | 4.6 | - |
| EX13NS6 | 5.9 | 11 | - |
| EX16NS6 | 0.25 | 1.9 | - |
| EX17NS6 | 0.10 | 1.2 | B |
| EX20NS6 | 0.89 | 5.0 | B |
| EX21 NS6 | 0.10 | 1.1 | A |
| EX24NS6 | 0.07 | 0.85 | B |

[Tab 2-2]

| compound | In vitro activity [nM] | | In vivo activity duration |
|---|---|---|---|
| | $EC_{50}$ | $IC_{50}$ | |
| EX25NS6 | 1.7 | 19 | - |
| EX27NS6 | > 10 | > 100 | - |
| EX28NS6 | 0.09 | 0.87 | - |
| EX29NS6 | 0.18 | 1.3 | - |
| EX30NS6 | 0.11 | 1.6 | - |
| EX31NS6 | 0.11 | 1.0 | - |
| EX32NS6 | 0.11 | 1.5 | B |
| EX33NS6 | 0.084 | 0.99 | B |
| EX34NS6 | 0.083 | 0.96 | B |

(continued)

| compound | In vitro activity [nM] | | In vivo activity duration |
|---|---|---|---|
| | $EC_{50}$ | $IC_{50}$ | |
| EX35NS6 | 0.085 | 0.72 | A |
| EX36NS6 | 0.085 | 0.72 | B |
| EX37NS6 | 0.14 | 0.99 | B |
| EX38NS6 | 0.10 | 0.84 | - |
| EX39NS6 | 0.094 | 0.86 | B |
| EX40NS6 | 0.05 | 0.86 | A |
| EX28NL | 0.039 | 0.52 | A |
| EX28NS3 | 0.029 | 0.49 | A |
| EX28NS36 | 0.036 | 0.72 | B |
| EX2840NG | 0.18 | 1 | - |
| EX2840NS6 | 0.06 | 1.3 | A |
| EX17212840NG | 0.13 | 1.5 | - |
| EX17213540NG | 0.11 | 1.3 | - |
| EX17283540NG | 0.061 | 0.73 | - |
| EX21283540NG | 0.092 | 1.1 | - |
| EX(1-28) | 0.074 | 0.99 | B |
| EX(1-28)28NG | 0.069 | 0.63 | A |
| EX(1-28)28NS6 | 0.099 | 1.1 | A |
| EX28CGlc | 0.065 | 0.4 | - |
| EX28CGal | 0.039 | 0.57 | B |
| EX28CLac | 0.024 | 0.41 | B |
| EX28CGen | 0.032 | 0.52 | B |
| EX28CSLac6 | 0.040 | 0.46 | B |
| EX28C7M | 0.047 | 0.62 | - |
| EX28CE1 | 0.091 | 3.2 | - |
| EX2840C7M | 0.085 | 0.76 | - |
| EX21283540C | 0.055 | 0.49 | B |
| EX21283540CGlc | 0.049 | 0.49 | - |
| EX21283540CGal | 0.049 | 0.53 | - |
| EX21283540CLac | 0.050 | 0.60 | - |
| EX21283540CGen | 0.052 | 0.65 | A |
| EX21283540CSLac6 | 0.26 | 3.7 | - |
| EX21283540C7M | 0.10 | 1.7 | - |
| EX28CJ3Gal | 0.034 | 0.41 | A |
| EX21283540CJ3Gal | 0.28 | 2.4 | - |

The result shows that a glycosylated peptide having glycochain at a position of 20 or later is preferable.

The present invention provides a pharmaceutical composition comprising a glycosylated GLP-1 related peptide or a pharmaceutically acceptable salt thereof, a diluetnt and an excipient. The pharmaceutical composition is usually prepared in the common manner of the pharmaceutical field and preferably administered parenterally. Examples of especially preferable route of administration include intramuscular and subcutaneous administrations. Dosage of the glycosylated peptide a day is in the range of about 1 pg/kg body weight to about 1000 μg/kg body weight, but more or less dosage is also effective. The necessary dosage depends on condition of disease, body length, body weight, gender, age and/or past medical history of a patient.

The pharmaceutical composition of the present invention can be prepared according to the conventional method, for example, description of Remington: Pharmacentical Science, 1985, or Remington: The Science and Practice of Pharmacy, 19th edition, 1995.

For example, a composition for infusion comprising the GLP-1 derivative of the present invention can be prepared to provide a requested final product by using the conventional technique in the pharmaceutical inductry to dissolve and mix the ingredients appropriately.

In order to prepare the composition of the present invention, an effective ingredient(comrising a sort of glycosylated GLP-1 related peptide at least) is usually mixed with or diluted with an excipient. Before mixing with the other ingredients, the glycosylated GLP-1 related peptide may be crushed to a powder having a suitable diameter.
Examples of the excipient include lactose, dextrose, sucrose, trehalose, sorbitol, mannitol, starch, arabia gum, calcium silicate, microcrystalline cellulose, polyvinyl pyrrolidone, cellulose, water, syrup, methyl cellulose and the like. Further, a lubricant such as talc, magnesium stearate and mineral oil; a wetting agent, an emulsifying agent, a suspension agent, a preservative such as methyl or propylhydroxy benzoic acid; a sweetner and a flavouring agent.

Usually, a pharmaceutical composition is prepared in a dosage unit comprising an effective ingredient of about 50 μg to 100 mg, preferably about 1 mg to about 10 mg.
According to a procedure, a GLP-1 derivative may be disssolved in a somewhat smaller amout of water than that of the final volume of the composition. If necessary, an isotonic agent, a preservative agent and a buffer solution may be added, and the pH may be adjusted by adding an acid such as hydrochloric acid, or a base such as a sodium hydrixide aq. solution. Finally, the volume of solution is adjusted by adding water and a requested concentration of the ingredient will be provided. A composition for nasal administration comprising a specified peptide may be prepared according to the description of EP 272097(Novo Nordisk A/S) or WO 93/18785.

In one aspect of the present invention, use of the GLP-1 derivative set forth above in manufacturing a pharmaceutical composition, especially the same for treating diabetes is provided.
In other aspect, a method for treating diabetes comprising an administration of the GLP-1 derivative set forth above.

Examples

In the present specification, a peptide and its glycosylated derivative may be shown by abbreviations, and the nomenclature is described by example 1 and 2 below.

example 1

[N(α-2,6- sialyl -LacNAc)34]-GLP-1

↓

GL34NS6

(1) (2) (3)

A part of (1) to (3) in the abbreviation"GL34NS6" means as follows;

(1) means a sort of peptide;

| | |
|---|---|
| GL: | GLP-1(7-36) amide |
| GLSGSGSG: | peptide of the above GL having an additional SGSGSG(amide) at the C terminus |
| EX: | Excendin-4 |
| EX(1-28): | Excendin-4(1-28) amide |

(2) means amino acid variation;

| | |
|---|---|
| 34N: | means amino acid$^{34}$ was replaced with Asn, |
| 28C: | means amino acid$^{28}$ was replaced with Cys, |

(continued)

| | |
|---|---|
| 01N: | means amino acid[1] was replaced with Asn, |
| 3437N: | means amino acid[34] and amino acid[37] were replaced with Asn respectively, |
| -1N: | means Asn was added at the N-terminus. |

(3) means a type of glycosilation as follows;

| | |
|---|---|
| G: | GlcNac |
| L: | LacNac |
| S3: | sialyl-α-2,3 LacNAc |
| S36: | disialyl LacNAc |
| E1: | branched N-glyco chain |
| E2: | branched N-glyco chain aggregate |
| 7M: | maltoheptaose |
| SLac3: | sialyl α-2,3 Lac |
| SLac6: | sialyl α-2,6 Lac |
| J3Gal: | Gal aggregate |

example 2: GL3437NS3

34 37

HAEGTFTSDVSSYLEGQAAKEFIAWLVKGR $-NH_2$

"GL3437NS3" means a glycosylated peptide in which Asn sialylα-2,3 LacNAc was introduced in 34- and 37-position of GLP-1(7-36) amide.

Example 1: Synthesis of 34-glycosylated GLP-1

(1) Preparation of GL34N and GL34NG

GL34N and GL34NG were prepared by solid phase peptide synthesis using Boc method or Fmoc method, and the products were purified with HPLC having an ODS column and lyophilized.

(2) Preparation of GL34NL

GL34NG(2 mM), UDP-Galactose(5 mM) and β-1,4-galactosyl transferase(0.2 U/mL, TOYOBO) were reacted in a solution(10 mM $MnCl_2$,12.5 mM HEPES buffer pH 7.5) at 25°C hor 2 hours. The reaction solution was concentrated by lyophilization and the product was purified with ODS column(Inertsil ODS-3 10x250 mm, GL Science) using 25 mM ammonium acetate-acetonitrile as an eluent.

(3) Preparation of GL34NS6

GL34NG(2 mM), UDP-Galactose(5 mM) and β-1,4-galactosyl transferase(0.2 U/mL, TOYOBO) were reacted in a solution(10 mM $MnCl_2$, 12.5 mM HEPES buffer pH 7.5, 500μl) at 25°C for 2 hours. The reaction solution was concentrated by lyophilization and a solution of 10 mM CMP-sialic acid, 50 mU/mL α 2,6-sialyl transferase(TOYOBO) and 0.01% Triton X-100 was finaly prepared by adding necessary agents. After the reacting volume was adjusted to 400 μl by adding water, the mixture was reacted at 37°C for 23 hours. During the reaction, 100 mM CMP-sialic acid(40

μl) was added. The product was purified with ODS column(Inertsil ODS-3 10x250 mm, GL Science) and 25 mM ammonium acetate-acetonitrile as an eluent.

(4) Preparation of GL34NE 1

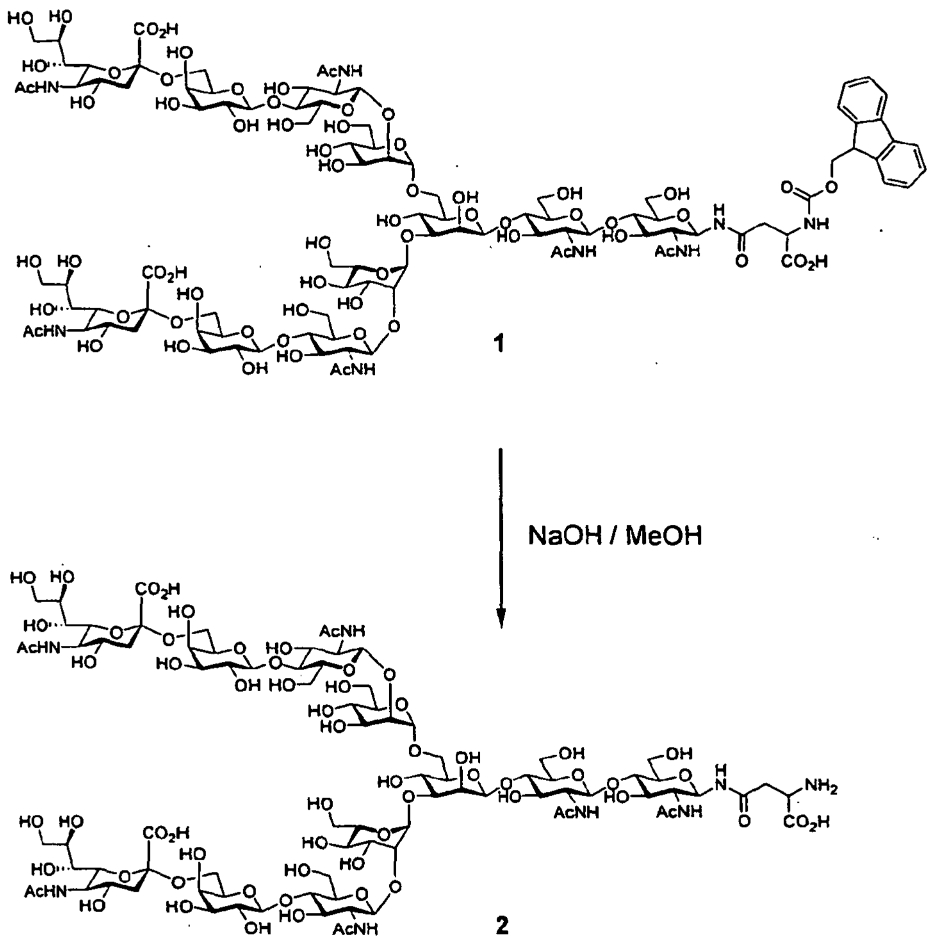


1

2

The compound(1)(150 mg, 58.6 μmol: Otsuka Cemical Co., Ltd.) was dissolved in methanol(60 mL) and a 1N sodium hydroxide aq. solution(1.8 mL) was added. After stirring at room temperature for 10 hours, the reaction was stopped by adding a 1N acetic acid aq, solution(3.6 mL). Methanol was evaporated, water and diethyl ether were added to the residue, and the aq. layer was washed with diethyl ether twice. A crude product of the compound(2) was obtained by lyophilization of the aq.layer, and then it was purified with a gel filtration chromatography(Sephadex G-50, mobile phase: water) to give the compound(2)(118 mg, 50.5 μmol), which was identified by MALDI-TOF-MS. MALDI-TOF-MS:[M (average)+Na]$^+$=2360.0, (theoretical value: [M(average)+Na]$^+$=2361.1)

A reaction solution(60 mM potassium phosphate buffer pH 6.25) including GL34NL(10 mM), the compound(2)(75 mM) and endo-β-N-acetylglucosaminidase (60mU/mL, Tokyo Chemical Industry Co., Ltd.) was reacted at 37°C for 2 hours, then the reaction was stopped by adding an equal amount of 8M guanidine hydrochloride solution and the product was purified with a reversed phase HPLC.

Example 2 . Synthesis of 37-glycosylated GLP-1

(1) Preparation of GL37N and GL37NG

GL37N and GL37NG were prepared by solid phase peptide synthesis using Boc method or Fmoc method, and the products were purified with HPLC having an ODS column and lyophilized.

(2) Preparation of GL37NL

GL37NG(1 mM), UDP-Galactose(3 mM) and β-1,4-galactosyl transferase(0.2 U/mL, TOYOBO) were reacted in a solution(10 mM $MnCl_2$,12.5 mM HEPES buffer pH 7.5 2mL) at 25°C hor 2 hours. The reaction solution was concentrated by lyophilization and the product was purified with a reversed phase HPLC.

(3) preparation of GL37NS6

GL37NG(1 mM), UDP-Galactose(3 mM) and β 1,4-galactosyl transferase(0.2 U/mL, TOYOBO) were reacted in a reaction solution(10 mM $MnCl_2$, 12.5 mM HEPES buffer pH 7.5, 1 mL) at 25°C for 2 hours. Then 100 mM CMP-sialic acid(50 μl), 1 U/mL α 2,6-sialyl transferase(TOYOBO)(50 μl) and 1 % Triron X-100(10 μl) were added and the mixture was reacted at 25°C for 26 hours. Further it was reacted at 37°C for 39 hours. During the reaction, 100 mM CMP-sialic acid(50 μl) and 1 U/mL α 2,6-sialyl transferase(TOYOBO)(25 μl) were added. The product was concentrated with lyophilization and purified with a reversed phase HPLC.

(4) Preparation of GL37NS3

GL37NG(2 mM), UDP-Galactose(5 mM) and β 1,4-galactosyl transferase(0.2 U/mL, TOYOBO) were reacted in a reaction solution(5 mM $MnCl_2$, 12.5 mM HEPES buffer pH 7.5, 1.4 mL) at 25°C for 2 hours, purified with a reversed phase HPLC and lyophilized. The GL37NL obtained in the above procedure was dissolved again in distilled water, and a reaction solution(50 mM HEPES buffer pH 7.5, 0.01 Triron X-100, 2 mL) containing GL37NL(1 mM), CMP-sialic acid (5 mM) and α 2,6-sialyl transferase(50 mU/mL, CALBIOCHEM) was prepared and it was reacted at 37°C for 0.5 hours. Then it was concentrated with lyophilization and purified with a reversed phase HPLC.

(5) Preparation of GL37NS36

A reaction solution(5 mM $MnCl_2$, 20 mM cacodylic acid buffer pH 7.0, 2 mL) containing GL37NS3(1 mM), CMP-sialic acid(5 mM) and α 2,6-sialyl transferase(JAPAN TOBACCO INC )(50mU/mL) was reacted at 30°C for 16 hours and the product was purified with a reversed phase HPLC and lyophilized.
(6) A reaction solution(60 mM potassium phosphate buffer pH 6.25) containing GL37NL(10 mM), the compound(2)(75 mM) and endo-β-N-acetylglucosaminidase(60 mU/mL, Tokyo Chemical Industry Co., Ltd.) was reacted at 37°C for 2 hours, then the reaction was stopped by adding an equal amount of 8M guanidine hydrochloride solution and the product was purified with a reversed phase HPLC.

Example 3 Preparation of other glycosylated GLP-1

The following glycosylated GLP-1's were prepared in the same manner as Examples 1 and 2:

GL08N, GL08NG, GL08NL, GL08NS6, GL08NE1, GL19N, GL19NG, GL19NL, GL19NS6, GL19NE1, GL26N, GL26NG, GL26NL, GL26NS6, GL26NE1, GLSGSGSG43NG, GLSGSGSG43NL and GLSGSGSG43NS6.

MS spectrum data were shown in Tables 3.
[Tab 3]

| Example | cmpound | theoretical figure (MW) | measured value (MW) | ionization method |
|---|---|---|---|---|
| 1(1) | GL34N | 3283.6 | 3283.8 | MALDI |
| 1(1) | GL34NG | 3486.8 | 3486.8 | MALDI |
| 1(2) | GL34NL | 3649.0 | 3649.0 | MALDI |
| 1(3) | GL34NS6 | 3940.2 | 3940.1 | MALDI |
| 1(4) | GL34NE1 | 5489.7 | 5489.7 | MALDI |
| 2(1) | GL37N | 3411.8 | 3411.8 | MALDI |
| 2(1) | GL37NG | 3615.0 | 3615.1 | MALDI |

(continued)

| Example | cmpound | theoretical figure (MW) | measured value (MW) | ionization method |
|---|---|---|---|---|
| 2(2) | GL37NL | 3777.1 | 3777.7 | MALDI |
| 2(3) | GL37NS6 | 4068.4 | 4068.4 | MALDI |
| 2(4) | GL37NS3 | 4068.4 | 4067.8 | ESI |
| 2(5) | GL37NS36 | 4359.7 | 4359.0 | ESI |
| 2(6) | GL37NE1 | 5617.8 | 5617.9 | MALDI |
| 3 | GL08N | 3340.7 | 3340.3 | MALDI |
| 3 | GL08NG | 3543.9 | 3544.0 | MALDI |
| 3 | GL08NL | 3706.1 | 3706.2 | MALDI |
| 3 | GL08NS6 | 3997.3 | 3997.9 | MALDI |
| 3 | GL08NE1 | 5546.7 | 5546.4 | MALDI |
| 3 | GL19N | 3248.6 | 3248.4 | MALDI |
| 3 | GL19NG | 3451.8 | 3451.7 | MALDI |
| 3 | GL19NL | 3614.0 | 3613.5 | MALDI |
| 3 | GL19NS6 | 3905.2 | 3905.1 | MALDI |
| 3 | GL19NE1 | 5454.6 | 5454.9 | MALDI |
| 3 | GL26N | 3283.6 | 3283.5 | MALDI |
| 3 | GL26NG | 3486.8 | 3486.9 | MALDI |
| 3 | GL26NL | 3649.0 | 3648.9 | MALDI |
| 3 | GL26NS6 | 3940.2 | 3939.8 | MALDI |
| 3 | GL26NE1 | 5489.7 | 5489.0 | MALDI |
| 3 | GLSGSGSG43NG | 4047.4 | 4045.6 | MALDI |
| 3 | GLSGSGSG43NL | 4209.5 | 4208.5 | MALDI |
| 3 | GLSGSGSG43NS6 | 4500.8 | 4501.2 | MALDI |
| ESI: Electrospray ionization Method<br>MALDI: Matrix Assisted Laser Desorption/Ionization Method | | | | |

Example 4 Synthesis of 34- and 37-glycosylated GLP-1

(1) Preparation of GL3437NG

GL3437NG was prepared by solid phase peptide synthesis using Boc method or Fmoc method, and the products were purified with HPLC having an ODS column and lyophilized.

(2) Preparation of GL3437NL

GL3437NG(2 mM), UDP-Galactose(6 mM), β 1,4-galactosyl transferase(0.2 U/mL, TOYOBO) and $MnCl_2$(10 mM) were reacted in a solution(25 mM HEPES buffer pH 7.5) at 25°C hor 16 hours and the product was purified with ODS column(Inertsil ODS-3 10x250 mm, GL Science) and 25 mM ammonium acetate-acetonitrile as an eluent.

(3) Preparation of GL3437NS6

A reaction solution(25 mM HEPES buffer pH 7.5) containing GL3437NL(1 mM), CMP-sialic acid (10 mM), 50 mU/mL α 2,6-sialyl transferase(0.1 U/mL, TOYOBO) and 0.01 % Triton X-100 was reacted at 37°C for 14 hours. The product was purified with ODS column(Inertsil ODS-3 10x250 mm, GL Science) and 25 mM ammonium acetate-acetonitrile as an eluent.

(4) Preparation of GL3437NS3

A reaction solution(50 mM HEPES buffer pH 7.5) containing GL3437NL(1 mM), CMP-sialic acid (10 mM), α 2,3-sialyl transferase(0.05 U/mL, Calbiochem) and 0.01% Triton X-100 was reacted at 37°C for 3.5 hours. Then, the product was purified with Inertsil ODS-3 10x250 mm(GL Science) using 25 mM ammonium acetate-acetonitrile as an eluent.

(5) Preparation of GL3437NS36

A reaction solution(5 mM $MnCl_2$, 20 mM cacodylic acid buffer pH 7.0, 0.44 mL) containing GL3437NS3(1 mM), CMP-sialic acid(10 mM) and α 2,6-sialyl transferase(50 mU/mL, JAPAN TOBACCO INC)was reacted at 30°C for 16 hours and the product was purified with a reversed phase HPLC and lyophilized.

Example 5 Preparation of other glycosylated GLP-1

The following glycosylated pepetides were prepared in the same manner as Examples 4;
GL2634NG, GL2634NL, GL2634NS6, GL2637NG, GL2637NL, GL2637NS6, GL263437NG, GL263437NL and GL263437NS6.

Example 6 Synthesis of glycosylated peptide by modification of a side chain thiol group in Cys-variational GLP-1

(1) Preparation of GL34C and GL3437C

GL34C and GL3437C were prepared by solid phase peptide synthesis using Boc method or Fmoc method, and the products were purified with HPLC having an ODS column and lyophilized.

(2) Preparation of GL34CE1

A reaction solution(100 mM phosphate buffer pH 8.0) containing GL34C(0.5 mM) and the compound(3)(1 mM, 1.2 mL) was reacted at 37°C for 24 hours. The mixture was purified with a reversed phase HPLC(Inertsil ODS-3 10x250 mm, GL Science) using 0.1 % TFA aq. solution and 0.1 % TFA acetonitrile as an eluent to give GL34CE1.

(3) Preparation of GL3437CE1

A reaction solution(100 mM phosphate buffer pH 8.0) containing GL3437C(0.5 mM) and the compound(3)(1.5 mM, 0.85 mL) was reacted. An aq. solution of the compound(3)(5 mM, 0.1 mL) was added 10 hours later and the mixture was further reacted for 13 hours. The mixture was purified with a reversed phase HPLC(Inertsil ODS-3 10x250 mm, GL Science) using 0.1 % TFA aq. solution and 0.1 % TFA acetonitrile as an eluent to give GL3437CE1.

(4) Preparation of GL34CE2

A reaction solution(100 mM phosphate buffer pH 8.0) containing GL34C(2 mM) and the compound(7)(2.5 mM, 0.2 mL) was reacted at 37°C for 25.5 hours. The mixture was purified with a reversed phase HPLC(Inertsil ODS-3 10x250 mm, GL Science) using 0.1 % TFA aq. solution and 0.1 % TFA acetonitrile as an eluent to give GL34CE2.

(5) Preparation of GL3437CE2

A reaction solution(100 mM phosphate buffer pH 8.0) containing GL3437C(0.25 mM) and the compound(7)(1 mM, 30 μl) was reacted at 37°C and the formation of GL3437CE2 was confirmed by MALDI-TOF-MS.
MS spectrum data of the compounds obtained in Examples 4-6 were shown in Tables 4.

[Tab 4]

| Example | compound | theoretical figure (MW) | measured value (MW) | ionization method |
|---|---|---|---|---|
| 4(1) | GL3437NG | 3804.1 | 3803.9 | ESI |

(continued)

| Example | compound | theoretical figure (MW) | measured value (MW) | ionization method |
|---|---|---|---|---|
| 4(2) | GL3437NL | 4128.4 | 4129.3 | ESI |
| 4(3) | GL3437NS6 | 4710.9 | 4710.7 | ESI |
| 4(4) | GL3437NS3 | 4710.9 | 4710.7 | ESI |
| 4(5) | GL3437NS36 | 5293.5 | 5291.7 | ESI |
| 5 | GL2634NG | 3676.0 | 3675.6 | ESI |
| 5 | GL2634NL | 4000.2 | 4000.0 | ESI |
| 5 | GL2634NS6 | 4582.8 | 4582.2 | ESI |
| 5 | GL2637NG | 3804.1 | 3803.8 | ESI |
| 5 | GL2637NL | 4128.4 | 4127.9 | ESI |
| 5 | GL2637NS6 | 4710.9 | 4711.1 | ESI |
| 5 | GL263437NG | 3993.3 | 3993.0 | ESI |
| 5 | GL263437NL | 4479.7 | 4479.7 | ESI |
| 5 | GL263437NS6 | 5353.5 | 5352.3 | ESI |
| 6(1) | GL3437C | 3375.8 | 3375.5 | ESI |
| 6(1) | GL34C | 3272.7 | 3272.3 | ESI |
| 6(2) | GL34CE1 | 5650.8 | 5649.6 | ESI |
| 6(3) | GL3437CE1 | 8132.1 | 8129.2 | ESI |
| 6(4) | GL34CE2 | 8100.1 | 8095.5 | ESI |
| 6(5) | GL3437CE2 | 13030.4 | 13035.2 | MALDI |

Example 7 Synthesis of 28-glycosylated excendin-4

(1) Preparation of EX28NG

EX28NG was prepared by solid phase peptide synthesis using Boc method or Fmoc method, and the products were purified with HPLC having an ODS column and lyophilized.

(2) Preparation of EX28NL

A reaction solution(10 mM $MnCl_2$, 25 mM HEPES buffer pH 7.5) containing EX28NG(2 mM), UDP-Galactose (5 mM), β 1,4-galactosyl transferase(0.2 U/mL, TOYOBO) was reacted at 25°C for 3 hours and the product was purified with C30 column(Develosil RPAQUEOUS AR-5 10×250mm, NOMURA CHEMICAL CO., LDP.) using 25 mM ammonium acetate-acetonitrile as an eluent.

(3) Preparation of EX28NS6

A reaction solution(10 mM $MnCl_2$, 12.5 mM HEPES buffer pH 7.5) containing EX28NG(1 mM), UDP-Galactose (5 mM) and β 1,4-galactosyl transferase(0.1 U/mL, TOYOBO) was reacted at 25°C for 2 hours. One tenth amount of 100 mM CMP-sialic acid and one tenth amount of 1 U/mL α 2,6-sialyl transferase(TOYOBO) were added and the solution was reacted at 37°C for 19 hours, and the product was purified with ODS colunm(Inertsil, ODS-3 10×250mm, GL Science).

(4) Preparation of EX28NS3.

A reaction solution(50 mM HEPES buffer pH 7.5) containing EX28NL(1 mM), CMP-sialic acid (10 mM), α 2,3-sialyl transferase(0.05 U/mL, Calbiochem) and 0.01 % Triton X-100 was reacted at 37°C for 17 hours. Then, the product was purified with C30 column RPAQUEOUS AR-5 10×250mm(NOMURA CHEMICAL CO., LDP.) using 25 mM ammonium acetate-acetonitrile as an eluent.

(5) Preparation of EX28NS36

A reaction solution(50 mM HEPES buffer pH 7.5) containing EX28NS3(1 mM), CMP-sialic acid(20 mM) and α 2,6-sialyl transferase(0.2 U/mL, JAPAN TOBACCO INC) was reacted at 30°C for 70 hours and the product was purified with C30 column RPAQUEOUS AR-5 10×250mm(NOMURA CHEMICAL CO., LDP.).

Example 8 Preparation of other glycosylated excendin-4

The following glycosylated pepetides were prepared in the same manner as Examples 7;
EX-1NG, EX01NG, EX02NG, EX03NG, EX04NG, EX05NG, EX06NG, EX07NG, EX08NG, EX09NG, EX10NG, EX11NG;
EX12NG, EX12NL, EX12NS6;
EX13NG, EX13NL, EX13NS6;
EX14NG, EX15NG,
EX16NG, EX16NL, EX16NS6;
EX17NG, EX17NL, EX17NS6;
EX18NG, EX19NG,
**[0065]** EX20NG, EX20NL, EX20NS6;
EX21NG, EX21NL, EX21NS6;
EX22NG, EX23NG,
EX24NG, EX24NL, EX24NS6;
EX25NG, EX25NL, EX25NS6;
EX26NG,
EX27NG, EX27NL, EX27NS6;
EX29NG, EX29NL, EX29NS6;
EX30NG, EX30NL, EX30NS6;
EX31NG, EX31NL, EX31NS6;
EX32NG, EX32NL, EX32NS6;
EX33NG, EX33NL, EX33NS6;
EX34NG, EX34NL, EX34NS6;
EX35NG, EX35NL, EX35NS6;
EX36NG, EX36NL, EX36NS6;
EX37NG, EX37NL, EX37NS6;
EX38NG, EX38NL, EX38NS6;
EX39NG, EX39NL, EX39NS6;
EX40NG, EX40NL, EX40NS6;
EX2840NG, EX2840NL, EX2840NS6;
EX17212840NG, EX17213540NG, EX17283540NG, EX21283540NG;
EX(1-28).
EX(1-28)28NG, EX(1-28)28NL, EX(1-28)28NS6.

Example 9 Synthesis of glycosylated peptide by modification of a side chain thiol group in Cys-variational excendin-4

(1) Preparation of EX28C and EX21283540C

EX28C and EX21283540C were prepared by solid phase peptide synthesis using Boc method or Fmoc method, and the products were purified with HPLC having an ODS column and lyophilized.

(2) Preparation of EX28CE1

A reaction solution(100 mM phosphate buffer pH 8.0) containing EX28C(1 mM) and the compound(3)(2 mM, 0.5 mL) was reacted at 37°C for 23 hours. The mixture was purified with a reversed phase HPLC(Inertsil ODS-3 10x250 mm, GL Science) to give EX28CE1.

(3) Preparation of EX28CJ3Gal

A reaction solution(100 mM phosphate buffer pH 8.0) containing EX28C(1 mM) and the compound(21)(2.7

mM, 0.36 mL) was reacted at 37°C for 3.5 hours. The mixture was purified with a reversed phase HPLC(Inertsil ODS-3 10x250 mm, GL Science) using 0.1 % TFA aq. solution and 0.1% TFA acetonitrile as an eluent to give EX28CJ3Gal.

(4) Preparation of EX21283540CJ3Gal

A reaction solution(100 mM phosphate buffer pH 8.0) containing EX21283540C(1 mM) and the compound(21) (8 mM, 0.23 mL) was reacted at 37°C. EX28C(0.5 mg) was added 2 hours later and the solution was further reacted for an hour. Then the mixture was purified with a reversed phase EPLC(Inertsil ODS-3 10x250 mm, GL Science) using 0.1 % TFA aq. solution and 0.1% TFA acetonitrile as an eluent to give EX21283540CJ3Gal.

(5) Preparation of EX28C7M

A reaction solution(100 mM phosphate buffer pH 8.0) containing EX28C(1 mM) and the compound(9)(4 mM, 0.40 mL) was reacted at 37°C for 3.5 hours and purified with C30 column RPAQUEOUS AR-5 10×250mm(NOMURA CHEMICAL CO., LDP.) to give EX28C7M.

(6) Preparation of EX21283540C7M

A reaction solution(100 mM phosphate buffer pH 8.0) containing EX21283540C(1 mM) and the compound(9) (8 mM, 0.37 mL) was reacted at 37°C for 1.5 hours and purified with C30 column RPAQUEOUS AR-5 10×250mm (NOMURA CHEMICAL CO., LDP.) to give EX21283540C7M.

(7) Preparation of other glycosylated peptides

The following glycosylated pepetides were prepared in the same manner as (5) and (6) above.
EX28CGlc, EX28CGal, EX28CLac, EX28CGen, EX2840C7M;
EX21283540CGlc, EX21283540CGal, EX21283540CLac, EX21283540Cgen.

(8) Preparation of EX28CSLac6

A reaction solution(50 mM Tris-HCl buffer pH 7.5) containing EX28CLac(0.5 mM), CMP-sialic acid(10 mM) and α 2,6-sialyl transferase(0.1 U/mL, JAPAN TOBACCO INC) and 0.01 % Triton X-100 was reacted at 16°C for 70 hours and the product was purified with ODS column Inertsil ODS-3 10x250 mm(GL Science) usung 25 mM ammonium acetate and acetonitrile as an eluent.

(9) Preparation of EX21283540CSLac6

A reaction solution(50 mM Tris-HCl buffer pH 7.5) containing EX21283540CLac(0.5 mM), CMP-sialic acid(10 mM) and α 2,6-sialyl transferase(0.1 U/mL, JAPAN TOBACCO INC) and 0.01 % Triton X-100 was reacted at 30°C for 16 hours and the product was purified with ODS column Inertsil ODS-3 10x250 mm(GL Science) usung 25 mM ammonium acetate and acetonitrile as an eluent.
MS spectrum data of the compounds obtained in Examples 6-9 were shown in
Tables 5.

| Example | compound | theoretical figure (MW) | measured value(MW) | ionization method |
|---|---|---|---|---|
| 7(1) | EX28NG | 4389.9 | 4388.8 | MALDI |
| 7(2) | EX28NL | 4552.0 | 4551.0 | MALDI |
| 7(3) | EX28NS6 | 4843.3 | 4843.9 | MALDI |
| 7(4) | EX28NS3 | 4843.3 | 4840.6 | ESI |
| 7(5) | EX28NS36 | 5134.5 | 5131.9 | ESI |
| 8 | EX-1NG | 4504.0 | 4502.8 | ESI |
| 8 | EX01NG | 4366.8 | 4366.4 | ESI |

(continued)

| Example | compound | theoretical figure (MW) | measured value(MW) | ionization method |
|---|---|---|---|---|
| 8 | EX02NG | 4446.9 | 4445.9 | ESI |
| 8 | EX03NG | 4374.8 | 4375.0 | ESI |
| 8 | EX04NG | 4446.9 | 4446.5 | ESI |
| 8 | EX05NG | 4402.9 | 4402.5 | ESI |
| 8 | EX06NG | 4356.8 | 4355.7 | ESI |
| 8 | EX07NG | 4402.9 | 4402.4 | ESI |
| 8 | EX08NG | 4416.9 | 4416.3 | ESI |
| 8 | EX09NG | 4388.9 | 4388.2 | ESI |
| 8 | EX10NG | 4390.8 | 4390.5 | ESI |
| 8 | EX11NG | 4416.9 | 4415.4 | ESI |
| 8 | EX12NG | 4375.8 | 4375.3 | ESI |
| 8 | EX12NL | 4537.9 | 4536.3 | ESI |
| 8 | EX12NS6 | 4829.2 | 4827.3 | ESI |
| 8 | EX13NG | 4375.8 | 4380.4 | ESI |
| 8 | EX13NL | 4538.0 | 4537.0 | ESI |
| 8 | EX13NS6 | 4829.2 | 4827.4 | ESI |
| 8 | EX14NG | 4372.8 | 4377.5 | ESI |
| 8 | EX15NG | 4374.8 | 4373.5 | ESI |
| 8 | EX16NG | 4374.8 | 4379.4 | ESI |
| 8 | EX16NL | 4537.0 | 4535.7 | ESI |
| 8 | EX16NS6 | 4828.2 | 4828.0 | ESI |
| 8 | EX17NG | 4374.8 | 4373.7 | ESI |
| 8 | EX17NL | 4537.0 | 4535.5 | ESI |
| 8 | EX17NS6 | 4828.2 | 4826.3 | ESI |
| 8 | EX18NG | 4432.9 | 4431.9 | ESI |
| 8 | EX19NG | 4404.8 | 4403.7 | ESI |

[Tab 6]

| Example | compound | theoretical figure (MW) | measured value(MW) | ionization method |
|---|---|---|---|---|
| 8 | EX20NG | 4347.8 | 4346.5 | ESI |
| 8 | EX20NL | 4509.9 | 4508.8 | ESI |
| 8 | EX20NS6 | 4801.2 | 4799.2 | ESI |
| 8 | EX21NG | 4390.8 | 4389.6 | ESI |
| 8 | EX21NL | 4552.9 | 4551.8 | ESI |
| 8 | EX21NS6 | 4844.2 | 4841.8 | ESI |
| 8 | EX22NG | 4356.8 | 4355.4 | ESI |
| 8 | EX23NG | 4390.8 | 4389.4 | ESI |

(continued)

| Example | compound | theoretical figure (MW) | measured value(MW) | ionization method |
|---|---|---|---|---|
| 8 | EX24NG | 4374.8 | 4373.6 | ESI |
| 8 | EX24NL | 4537.0 | 4535.6 | ESI |
| 8 | EX24NS6 | 4828.2 | 4826.3 | ESI |
| 8 | EX25NG | 4317.7 | 4316.5 | ESI |
| 8 | EX25NL | 4479.9 | 4478.5 | ESI |
| 8 | EX25NS6 | 4771.1 | 4769.2 | ESI |
| 8 | EX26NG | 4390.8 | 4390.3 | ESI |
| 8 | EX27NG | 4375.8 | 4375.6 | ESI |
| 8 | EX27NL | 4537.9 | 4537.2 | ESI |
| 8 | EX27NS6 | 4829.2 | 4827.5 | ESI |
| 8 | EX29NG | 4446.9 | 4446.3 | ESI |
| 8 | EX29NL | 4609.1 | 4607.6 | ESI |
| 8 | EX29NS6 | 4900.3 | 4898.4 | ESI |
| 8 | EX30NG | 4446.9 | 4445.9 | MALDI |
| 8 | EX30NL | 4609.1 | 4608 | MALDI |
| 8 | EX30NS6 | 4900.3 | 4900.6 | MALDI |
| 8 | EX31NG | 4406.8 | 4406.5 | ESI |
| 8 | EX31NL | 4569.0 | 4567.7 | ESI |
| 8 | EX31NS6 | 4860.2 | 4858.7 | ESI |
| 8 | EX32NG | 4416.9 | 4416.5 | ESI |
| 8 | EX32NL | 4579.0 | 4577.8 | ESI |
| 8 | EX32NS6 | 4870.3 | 4868.3 | ESI |
| 8 | EX33NG | 4416.9 | 4416.5 | ESI |
| 8 | EX33NL | 4579.0 | 4577.7 | ESI |
| 8 | EX33NS6 | 4870.3 | 4868.3 | ESI |
| 8 | EX34NG | 4446.9 | 4446.5 | ESI |
| 8 | EX34NL | 4609.1 | 4607.9 | ESI |
| 8 | EX34NS6 | 4900.3 | 4898.3 | ESI |
| 8 | EX35NG | 4432.9 | 4432.6 | ESI |
| 8 | EX35NL | 4595.0 | 4594.3 | ESI |
| 8 | EX35NS6 | 4886.3 | 4884.6 | ESI |

[Tab 7]

| Example | compound | theoretical figure (MW) | measured value(MW) | ionization method |
|---|---|---|---|---|
| 8 | EX2840NG | 4707.2 | 4706.1 | ESI |
| 8 | EX2840NL | 5031.4 | 5029.7 | ESI |
| 8 | EX2840NS6 | 5614.0 | 5611.6 | ESI |

(continued)

| Example | compound | theoretical figure (MW) | measured value(MW) | ionization method |
|---|---|---|---|---|
| 8 | EX17212840NG | 5099.5 | 5096.8 | ESI |
| 8 | EX17213540NG | 5142.5 | 5139.8 | ESI |
| 8 | EX17283540NG | 5141.6 | 5139.0 | ESI |
| 8 | EX21283540NG | 5157.5 | 5155.1 | ESI |
| 8 | EX(1-28) | 3294.7 | 3292.6 | ESI |
| 8 | EX(1-28)28NG | 3497.9 | 3498.8 | ESI |
| 8 | EX(1-28)28NL | 3660.1 | 3660.3 | ESI |
| 8 | EX(1-28)28NS6 | 3951.3 | 3949.3 | ESI |
| 9(1) | EX28C | 4175.7 | 4173.9 | ESI |
| 9(2) | EX28CE1 | 6553.9 | 6550.2 | ESI |
| 9(3) | EX28CJ3Gal | 5146.6 | 5144.2 | ESI |
| 9(4) | EX21283540CJ3Gal | 8184.4 | 8184.8 | ESI |
| 9(5) | EX28C7M | 5367.8 | 5367 | ESI |
| 9(6) | EX21283540C7M | 9069.1 | 9063 | ESI |
| 9(7) | EX28CGlc | 4394.9 | 4393 | ESI |
| 9(7) | EX28CGal | 4394.9 | 4393 | ESI |
| 9(7) | EX28CLac | 4557.0 | 4555 | ESI |
| 9(7) | EX28CGen | 4557.0 | 4555 | ESI |
| 9(7) | EX2840C7M | 6663.0 | 6657.9 | ESI |
| 9(7) | EX21283540C | 4300.9 | 4299.5 | ESI |
| 9(7) | EX21283540CGlc | 5177.7 | 5174.8 | ESI |
| 9(7) | EX21283540CGal | 5177.7 | 5175.1 | ESI |
| 9(7) | EX21283540CLac | 5826.2 | 5822.8 | ESI |
| 9(7) | EX21283540CGen | 5826.2 | 5823.1 | ESI |
| 9(8) | EX28CSLac6 | 4848.3 | 4854.9 | ESI |
| 9(9) | EX21283540CSLac6 | 6991.3 | 6996.9 | ESI |

A method for preparing a reagent used for glycosylation reaction.

Reference Example 1 Synthesis of the compound(3)

2

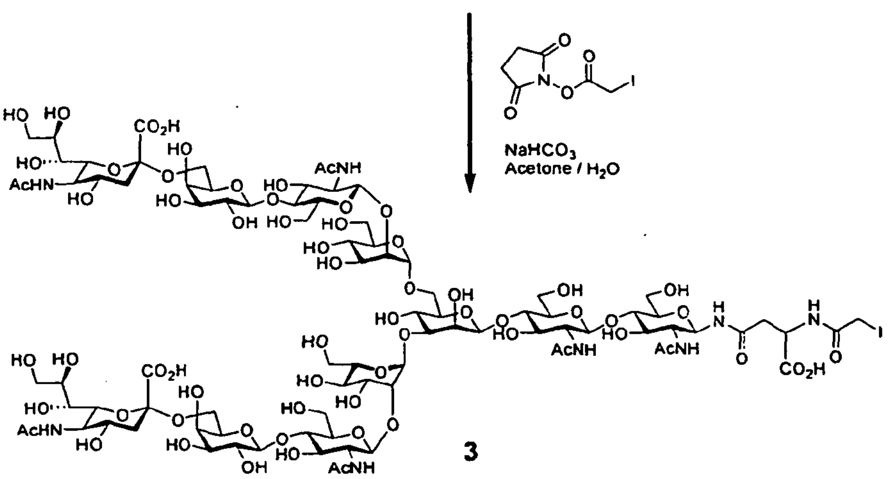


3

An aqueous solution(5 mL) containing the compound(2)(2 mM), iodoacetic acid N-hydroxysuccinimide ester (10 mM), sodium bicarbonate(15 mM) and 50 %(v/v) acetone was reacted at room temperature for 1.5 hours and the reacton was stopped by adding ammonia water(100 mM, 0.5 mL) The reaction solution was neutralized by adding a 1N acetic acid aq. solution and acetone was evaporated in vacuo. The residue was purified with a reversed phase HPLC (Inertsil ODS-3 10×250mm, GL Science) usung 0.1% TFA aq. solution and 0.1 % TFA acetonitrile as an eluent, and a gel filtration chromatography(Sephadex G-15) using water as a mobile phase to give the compound(3)(4.1 mg). the product was identified with ESI-MS.
ESI-MS:$[M+2H]^{2+}$=1252.7, (theoretical value:$[M+2H]^{2+}$=1254.0).

Reference Example 2 Synthesis of the compound(4)

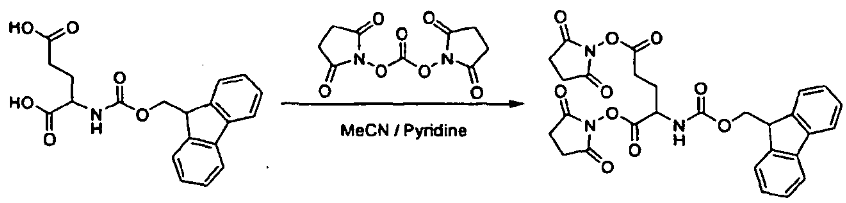


4

Fmoc-Glu-OH(189 mg), DSC(N,N'-Disuccinimidyl carbonate)(512 mg) and pyridine(158 mg) were dissolved in acetonitrile and heated to reflux for 5 hours. After being cooled to room temperature, acetonitrile was evaporated in vacuo. Ethyl acetate was added to the residue, the organic aolution was washed with 1N hydrochloric acid and brine, dried over magnesium sulfate and the solvent was evaporated in vacuo to give a mixture containing the compound(4) (0.36 g). It was used in the next step without further purification.
MALDI-TOF-MS:$[M(average)+Na]^+$=587.3, (theoretical value: $[M(average)+Na]^+$= 586.50),
$^1$H-NMR($CDCl_3$):δ7.77(d,2H), 7.61(br,2H), 7.41(t,2H), 7.32(t,7.32), 5.67(d,1H), 4.90(m,1H), 4.50-4.39(m,2H), 4.24(t, 1H), 2.84(br,8H), 2.95-2.65(m,2H), 2.47(m,1H), 2.36(m,1H).

Reference Example 3 Synthesis of the compound(5)

4

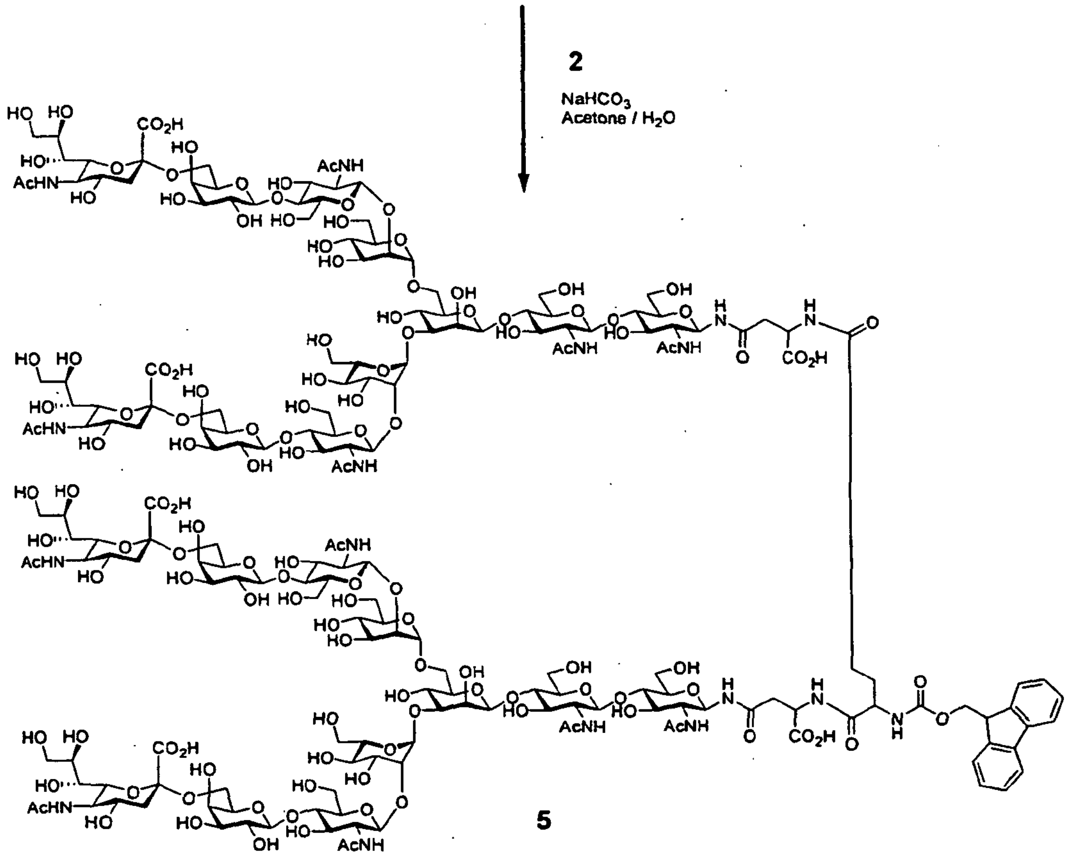


5

An aqueous solution(1 mL) containing the compound(2)(5 mM), the compound(4)(2 mM), sodium bicarbonate (10 mM) and 50%(v/v) acetone was reacted at room temperature for 3 hours. An aqueous solution of the compound(2) (20mM, 125 μl) was added and the solution was further reacted for 1.5 hours, and the reaction was stopped by adding ammonia water(100 mM, 50μl). After the solution was neutralized by adding a 1N acetic acid aq. solution, acetone was evaporated in vacuo and the residue was purified with a reversed phase HPLC(Inertsil ODS-3 10×250mm, GL Science) usung 0.1% TFA aq. solution and 0.1% TFA acetonitrile as an eluent to give the compound(5)(2.2 mg). MALDI-TOF-MS:$[M(average)+Na]^+$=5037.8, (theoretical value: $[M(average)+Na]^+$= 5032.5).

Reference Example 4 Synthesis of the compound(6)

5

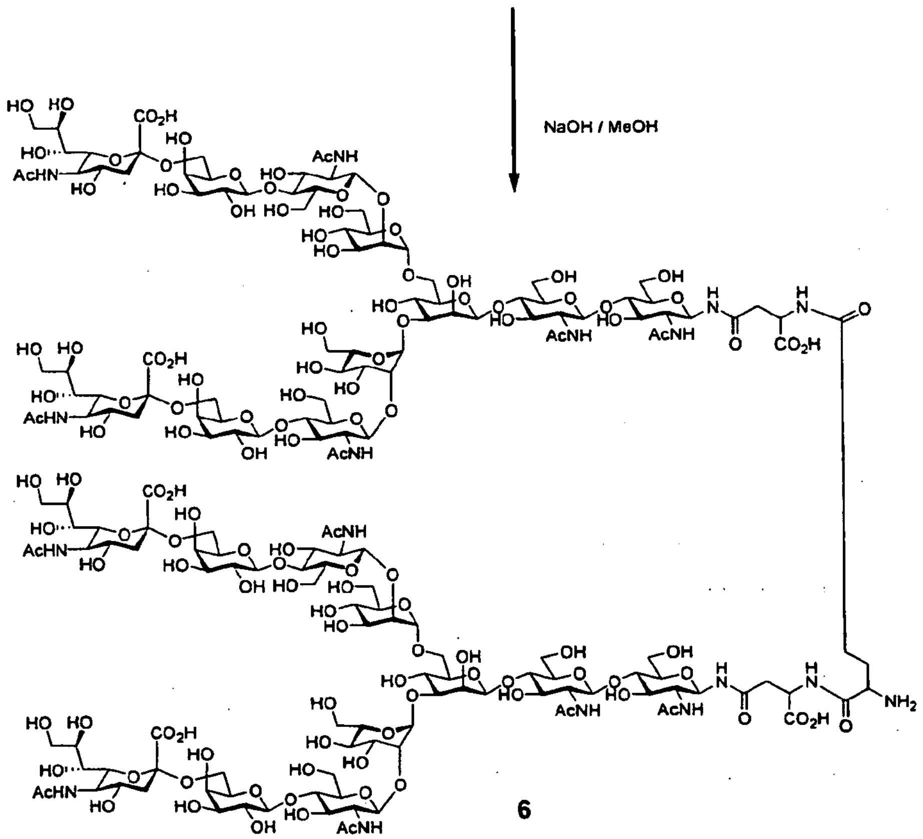


6

An aqueous solution(1.7 mL) containing the compound(5)(2.2 mg), sodium hydroxide(22 mM) and 88%(v/v) methanol was reacted at room temperature. A 1N sodium hydroxide aq. solution(15 μ.) was added and the solution was further reacted for 2 hours. The reaction was stopped by neutralization with a 1N acetic acid aq. solution, methanol was evaporated in vacuo and water was added to the residue and the aqueous solution was washed with diethyl ether twice. The washed aqueous layer was purified with a gel filtration chromatography(Sephadex G-15) using water as a mobile phase to give a mixture containing the compound(6)(2.4 mg). It was used in the next step without further purification.

Reference Example 5 Synthesis of the compound(7)

6

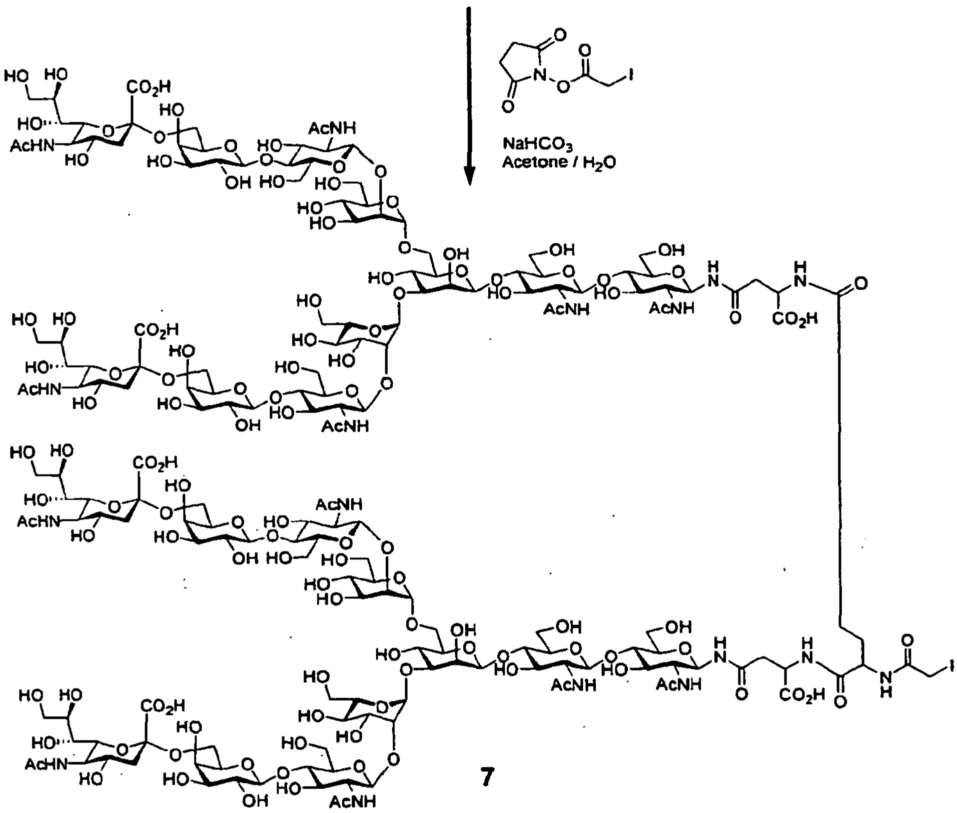


7

An aqueous solution(0.4 mL) containing the compound(6)(1 mM), iodoacetic acid N-hydroxysuccinimide ester (5 mM), sodium bicarbonate(10 mM) and 50 %(v/v) acetone was reacted at room temperature for 2 hours and the reacton was stopped by adding ammonia water(100 mM, 40 μl).
The reaction solution was neutralized by adding a 1N acetic acid aq. solution and acetone was evaporated in vacuo. The residue was purified with a reversed phase HPLC(Inertsil ODS-3 10×250mm, GL Science) usung 0.1% TFA aq. solution and 0.1% TFA acetonitrile as an eluent to give the compound(7)(1.5 mg). The product was identified with MALD-TOF-MS.
MALDI-TOF-MS:[M(average)+Na]$^+$=4979.7, (theoretical value :[M(average)+Na]$^+$= 4978.2).

Reference Example 6 Synthesis of the compound(9)

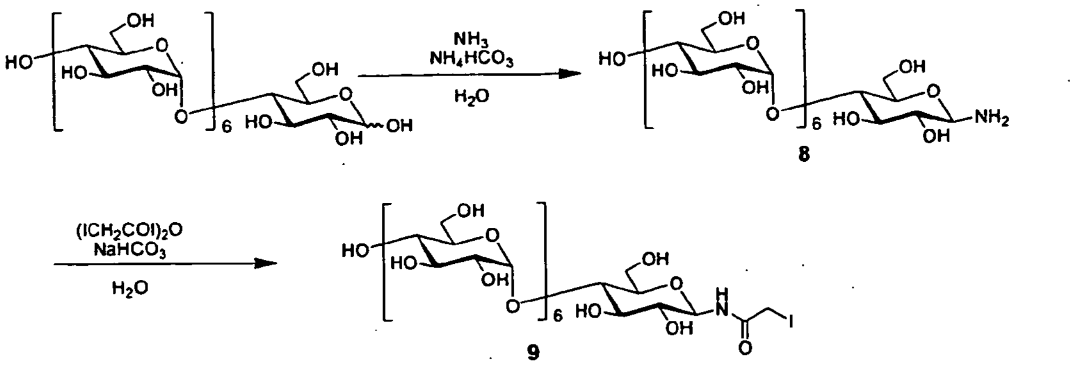


8

9

Sodium bicarbonate(1.6 mg) and maltheptaose(23 mg) were dissolved in 16M ammonia water(0.1 mL) and reacted at 42°C for 36 hours. After the reaction, the solution was concentrated in vacuo, lyophilized to give a mixture containing the compound(8). It was dissolved in a 1M sodium bicarbonate solution(0.2 mL), iodoacetic anhydride(35 mg) was added therein and the mixture was reacted at room temperature for an hour. Then the reaction was stopped

by adding 1M ammonia water(0.1 mL). The reaction solution was neutralized by acetic acid and purified with C30 column RPAQUEOUS AR-5 10×250mm(NOMURA CHEMICAL CO., LDP.) to give the compound(9)(4.5 mg).
ESI-MS:$[M+H]^+$=1320.1, (theoretical value: $[M+H]^+$=1320.3).

Reference Example 7 Synthesis of the other iodoacetyl glycoside.
The compound(10), (11), (12) and (13) were synthesized in the same manner as the compound(9).

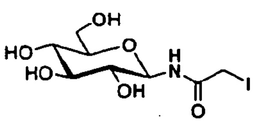

10

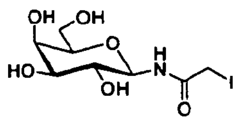

11

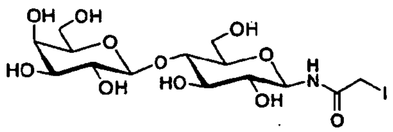

12

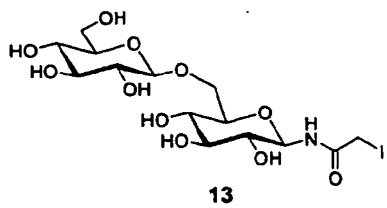

13

compound(10) ESI-MS:$[M+Na]^+$=369.8, (theoretical value:$[M+Na]^+$=370.0)
compound(11) ESI-MS:$[M+Na]^+$=369.8, (theoretical value:$[M+Na]^+$=370.0)
compound(12) ESI-MS:$[M+H]^+$=510.0, (theoretical value:$[M+H]^+$=510.1)
compound(13) ESI-MS:$[M+Na]^+$=532.1, (theoretical value:$[M+Na]^+$=532.0)

Reference Example 8 Synthesis of the compound(14)

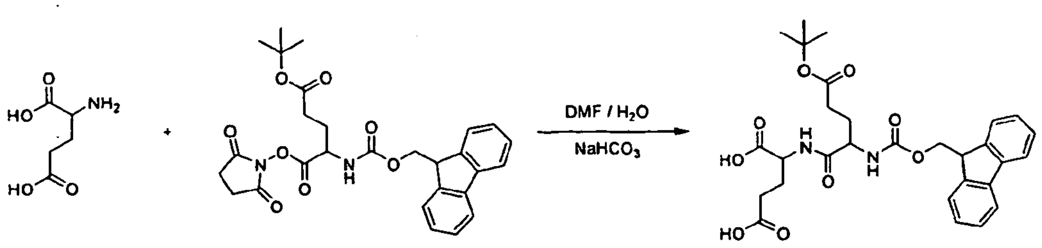


14

Sodium bicarbonate(3.6 g) and glutamic acid(2.1 g) were added to a mixture of water(50 mL) and DMF(20 mL) and cooled to under 10°C. A solution of Fmoc-Glu(OtBu)-OSu in DMF was poured into the solution and DMF(20 mL) was further added and the mixture was reacted at room temperature for 2 hours. After the reaction, the solution was acidified by adding 1N hydrochloric acid and extracted with ethyl acetate. The organic phase was washed with water, dried over magnesium sulfate and the solvent was evaporated in vacuo to give a mixture containing the compound(14) (1.6 g). It was used in the next step without further purification.
MALDI-TOF-MS: $[M(average)+Na]^+$=577.35, (theoretical value: $[M(average)+Na]^+$=577.58).

Reference Example 9 Synthesis of the compound(15)

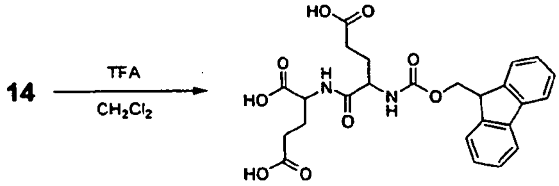


15

The mixture of the compound(14)(1.5 g) was dissolved in 50% TFA dichloromethane solution and the mixture was stirred

at room temperature for an hour. The solvent was evaporated in vacuo, diethyl ether was added to the residue to give the precipitate, which was filtered and dried to give a mixture containing the compound(15)(1.2 g). It was used in the next step without further purification.

MALDI-TOF-MS: $[M(average)+H]^+$=499.30, (theoretical value: $[M(average)+H]^+$=499.49).

Reference Example 10 Synthesis of the compound(16)

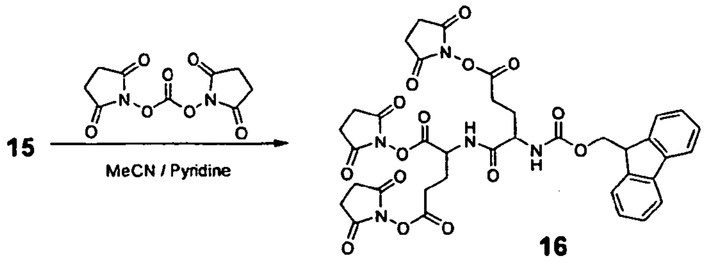


The compound(15)(0.71 g), DSC(N,N'-disuccinimidyl carbonate, 2.2 g) and pyridine(0.68 g) were dissolved in acetonitorile and heated under reflux for 10 hours. Then, the reaction solution was cooled to room temperature and acetonitrile was evaporated in vacuo. Ethyl acetate was added to the residue and the organic layer was washed with 1N hydrochloric acid and brine, dried over magnesium sulfate and the solvent was evaporated to give a mixture containing the compound(16)(0.92 g). It was used in the next step without further purification.

MALDI-TOF-MS: $[M(average)+Na]^+$=812.36, (theoretical value: $[M(average)+Na]^+$=812.69).

Reference Example 11 Synthesis of the compound(17)

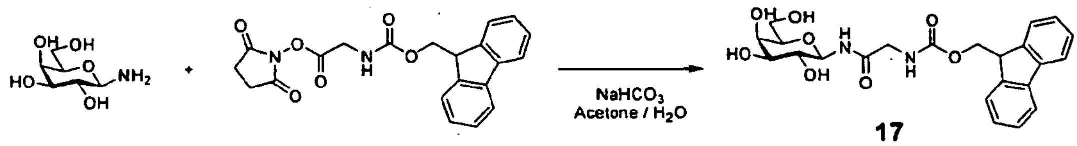


1-Amino-1-deoxy-β-D-galactose(175 mg) and sodium bicarbonate(336 mg) were dissolved in 50% acetone aq. solution(50 mL) and a solution of Fmoc-Gly-OSu(788 mg) in acetone was added therein. Acetone(20 mL) was further added and the mixture was stirred at room temperature for 3.5 hours. Then acetone was evaporated and the resulting aq.solution was purified with a reversed phase HPLC(YMC Pack, ODS-A 20×250mm) using 0.1 % TFA aq. solution and 0.1% TFA acetonitrile as an eluent to give the compound(17)(96 mg).

ESI-MS:$[M(average)+H]^+$=459.3, (theoretical value: $[M(average)+H]^+$= 459.5).

Reference Example 12 Synthesis of the compound(18) and (19)

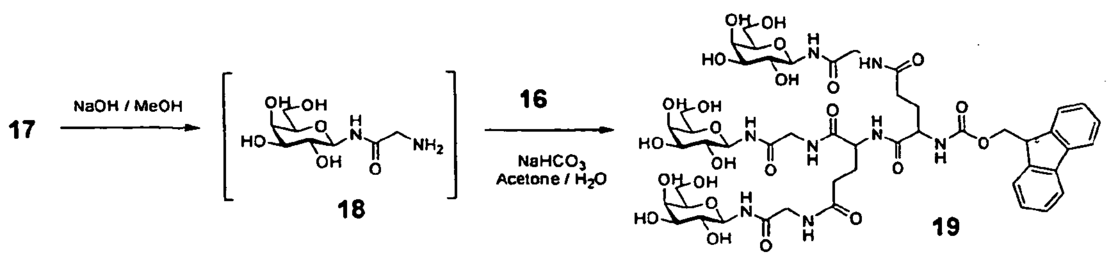


The compound(17)(60 mg) was dissolved in methanol(25 mL), adjusted to pH 12-13 by adding a 1N sodium hydroxide aq. solution and the mixture was reacted at room temperature for 3.5 hours. Then, the reaction solution was neutralized with acetic acid, methanol was evaporated in vacuo, water was added to the residue and the aq. solution was washed with diethyl ether. The aq. solution was concentrated in vacuo and remaining diethyl ether was removed to give an aq. solution(about 2 mL) containing the compound(18). Acetone(1 mL) was added and adjusted to pH 7.0-7.5 by adding a sodium bicarbonate aq. solution(500 mM) and a solution of the compound(16)(21 mg) in acetone was added. After stirring at room temperature for 0.5 hour, a solution of the compound(16)(8 mg) in acetone and the mixture was reacted

for additional 1 hour. After the reaction, the reaction solution was neutralized with acetic acid and acetone was evaporated in vacuo. The resulting ersidue was purified with a reversed phase HPLC(YMC Pack, ODS-A 20×250mm) using 0.1% TFA aq. solution and 0.1% TFA acetonitrile as an eluent to give the compound(19)(13 mg).
ESI-MS:[M(average)+H]$^+$=1153.7, (theoretical value: [M(average)+H]$^+$= 1154.1).

Reference Example 13 Synthesis of the compound(20) and (21)

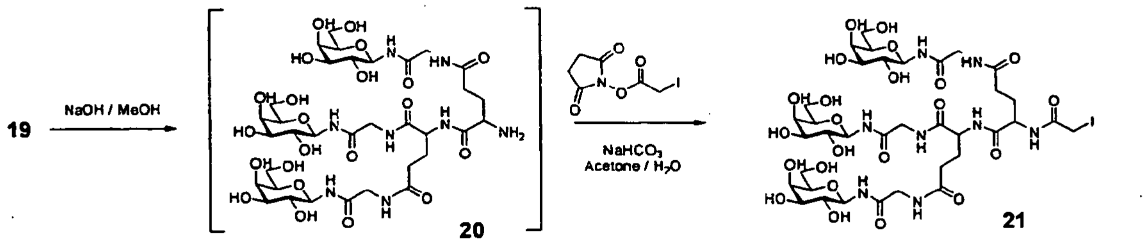


The compound(19)(13 mg) was dissolved in a 50% methanol aq. solution(6 mL) and the mixture was adjusted to pH 12-13 by adding a 1N sodium hydroxide aq.solution and reacted at room temperature for 2 hours. Then, the reaction solution was neutralized with acetic acid, methanol was evaporated in vacuo, water was added to the residue and the aq. solution was washed with diethyl ether. The aq. solution was concentrated in vacuo and remaining diethyl ether was removed to give an aq. solution(about 1.5 mL) containing the compound(20). Acetone(1 mL) was added to the resulting aq. solution, pH of the solution was adjusted to 7.0-7.5 with a sodium bicarbonate aq. solution(500 mM), and a solution of iodoacetic acid N-hydroxysuccimide ester(4.3 mg) in acetone was added and the mixture was reacted at room temperature for 0.5 hour. Then the reaction was stopped by adding ammonium acetate(500 mM, 40 μl) and neutralized with acetic acid. Acetone was evaporated and the residue was purified with a reversed phase HPLC(Inertsil ODS-3 10×250mm) using 0.1% TFA aq. solution and 0.1% TFA acetonitrileas an eluent to give the compound(21)(3 mg).
ESI-MS:[M(average)+H]$^+$=1099.4, (theoretical value: [N4(average)+H]$^+$= 1099.8).

## Claims

1. A glycosylated GLP-1 related peptide derivative of the formula;

| | | | | | | | | | | |
|---|---|---|---|---|---|---|---|---|---|---|
| 7 | 8 | 9 | 10 | 11 | 12 | 13 | 14 | 15 | 16 | 17 |
| His | Ala | Glu | Gly | Thr | Phe | Thr | Ser | Asp | Val | Ser |
| 18 | 19 | 20 | 21 | 22 | 23 | 24 | 25 | 26 | 27 | 28 |
| Ser | Tyr | Leu | Glu | Gly | Gln | Ala | Ala | Xpp | Glu | Phe |
| 29 | 30 | 31 | 32 | 33 | 34 | 35 | 36 | 37 | | |
| Ile | Ala | Trp | Leu | Val | Xvv | Gly | Arg | Xyy | | |

wherein Xpp is Lys or a glycosylated amino acid residue selected from the group A of glycosylated amino acid residues containing

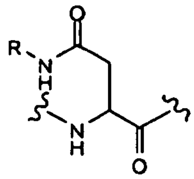
(c) ,

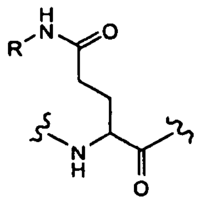
(d) ,

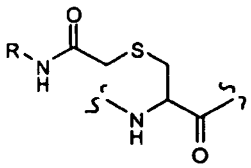
(g) ,

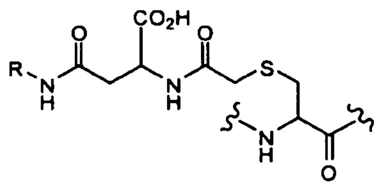
(g') .

or

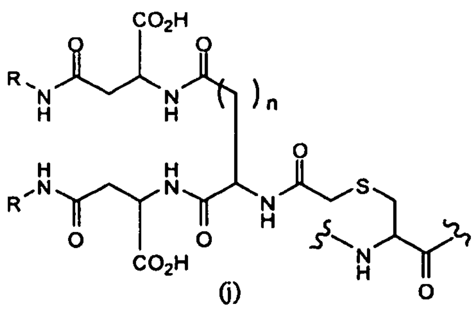
(j)

wherein n is an integer of 1 to 10, and R is the glycochain selected from
GlcNAc
Galβ1-4GlcNAc
NeuAcα2-6Galβ1-4GlcNAc
Galpl-4(Fucα1-3)GlcNAc
NeuAcα2-3Galβ1-4GlcNAc

NeuAcα2-3Galβ1-4(Fucα1-3)GlcNAc
NeuAcα2-6Galβ1-4(Fucα1-3)GlcNAc
GlcNAcβ1 (-3Galβ1-4G)cNAcβ1)$_n$-3Galβ1-4GlcNAc
Galβ1-4GlcNAcβ1(-3Galβ1-4GlcNAcβ1)$_n$-3Galβ1-4GlcNAc
NeuAcα2-3Galβ1-4GlcNAcβ1(-3Galβ1-4GlcNAcβ1)$_n$-3Galβ1-4GleNAc
NeuAcα2-6Galβ1-4GlcNAcp1(-3Galβ1-4GlcNAcβ1)$_n$-3Galβ1-4GlcNAc

NeuAcα2-6Galβ1-4GlcNAcβ1-2Manα1 6<br>Manβ1-4GlcNAcβ1-4GlcNAcβ<br>NeuAcα2-6Galβ1-4GlcNAcβ1-2Manα1 3

NeuAcα2-6Galβ1-4GlcNAcβ1-2Manα1 6<br>Manβ1-4GlcNAcβ1-4GlcNAcβ<br>Galβ1-4GlcNAcβ1-2Manα1 3

Galβ1-4GlcNAcβ1-2Manα1 6<br>Manβ1-4GlcNAcβ1-4GlcNAcβ<br>NeuAcα2-6Galβ1-4GlcNAcβ1-2Manα1 3

NeuAcα2-6Galβ1-4GlcNAcβ1-2Manα1 6<br>Manβ1-4GlcNAcβ1-4GlcNAcβ<br>GlcNAcβ1-2Manα1 3

GlcNAcβ1-2Manα1 6<br>Manβ1-4GlcNAcβ1-4GlcNAcβ<br>NeuAcα2-6Galβ1-4GlcNAcβ1-2Manα1 3

NeuAcα2-6Galβ1-4GlcNAcβ1-2Manα1 6<br>Manβ1-4GlcNAcβ1-4GlcNAcβ<br>Manα1 3

Manα1 6<br>Manβ1-4GlcNAcβ1-4GlcNAcβ<br>NeuAcα2-6Galβ1-4GlcNAcβ1-2Manα1 3

Galβ1-4GlcNAcβ1-2Manα1 6<br>Manβ1-4GlcNAcβ1-4GlcNAcβ<br>Galβ1-4GlcNAcβ1-2Manα1 3

GlcNAcβ1-2Manα1 6<br>Manβ1-4GlcNAcβ1-4GlcNAcβ<br>GlcNAcβ1-2Manα1 3

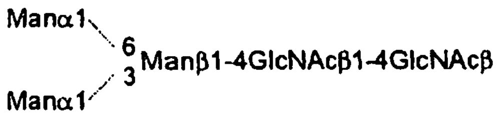

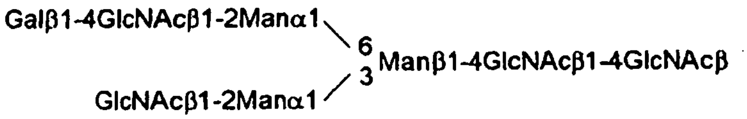

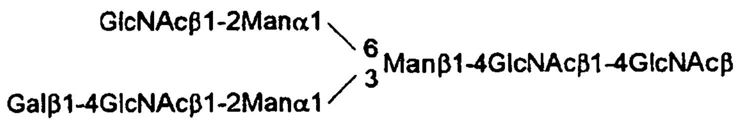

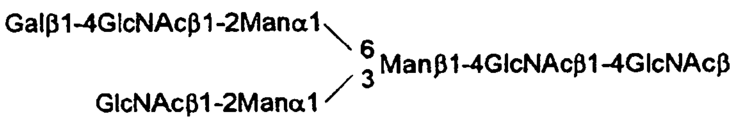

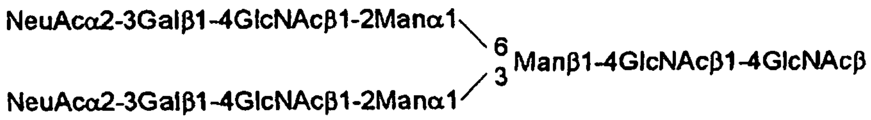

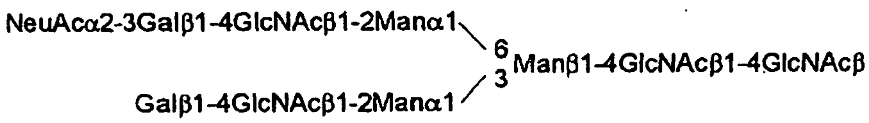

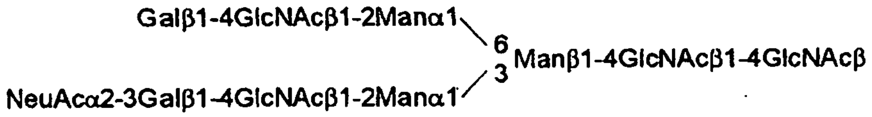

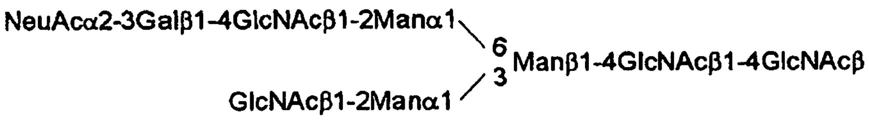

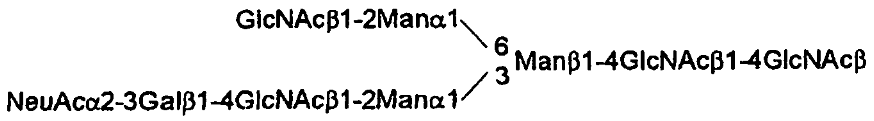

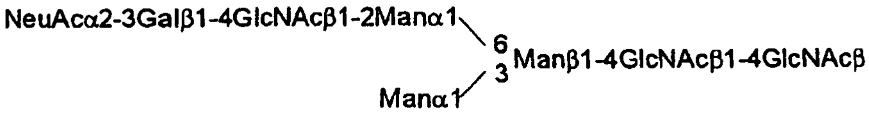

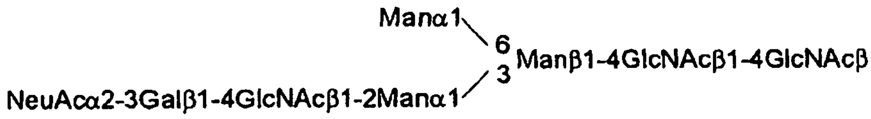

NeuAcα2-6(NeuAca2-3)Galβ1-4GlcNAc and Gen,
Xvv is Lys or a glycosylated amino acid residue selected from the group A of glycosylated amino acid residues,
Xyy is Gly or a glycosylated amino acid residue selected from the group A of glycosylated amino acid residues,
wherein one to three residue(s) of Xpp, Xvv and/or Xyy is (are) a glycosylated amino acid residue selected from the group A of glycosylated amino acid residues,
or its ester, amide, alkylamide or dialkylamide; and/or a pharmaceutically acceptable salt thereof.

**2.** A glycosylated GLP-1 related peptide derivative of the formula below;

| | | | | | | | | | | |
|---|---|---|---|---|---|---|---|---|---|---|
| 7 | 8 | 9 | 10 | 11 | 12 | 13 | 14 | 15 | 16 | 17 |
| His | Gly | Glu | Gly | Thr | Phe | Thr | Ser | Asp | Leu | Ser |
| 18 | 19 | 20 | 21 | 22 | 23 | 24 | 25 | 26 | 27 | 28 |
| Lys | Gln | Met | Glu | Glu | Glu | Ala | Val | Arg | Xqq | Phe |
| 29 | 30 | 31 | 32 | 33 | 34 | 35 | 36 | 37 | 38 | 39 |
| Ile | Glu | Trp | Leu | Lys | Xvv | Gly | Gly | Pro | Ser | Ser |
| 40 | 41 | 42 | 43 | 44 | 45 | | | | | |
| Gly | Ycc | Pro | Pro | Pro | Ser | | | | | |

wherein Xqq is Leu or a glycosylated amino acid residue selected from the group A of glycosylated amino acid residues containing

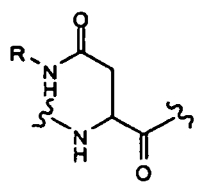

(c) ,

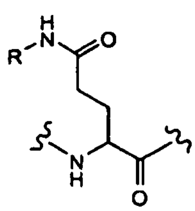

(d) ,

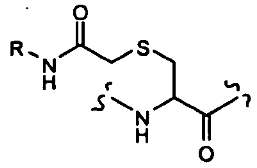

(g)

,

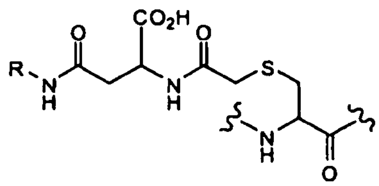

(g')

.

or

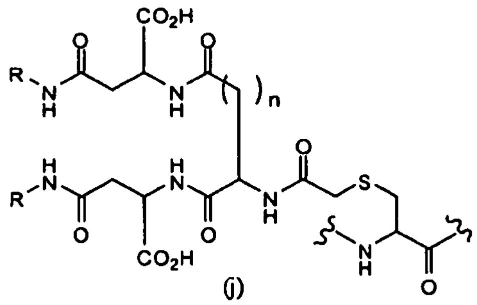

(j)

wherein n is an integer of 1 to 10, and R is the glycochain selected from
GlcNAc
Galβ1-4GlcNAc
NeuAcα2-6Galβ1-4GlcNAc
Galβ1-4(Fucα1-3)GlcNAc
NeuAcα2-3Galβ1-4GlcNAc
NeuAcα2-3Galβ1-4(Fucα1-3)GlcNAc
NeuAcα2-6Galβ1-4(Fucα1-3)GlcNAc
GleNAcβ1(-3Galβ1-4GlcNAcβ1)$_n$-3Galβ1-4GlcNAc
Galβ1-4GlcNAcβ1(-3Gaβp1-4GlcNAcβ1)$_n$-3Galβ1-4GlcNAc
NeuAcα2-3Galβ1-4GlcNAcβ1(-3Galβ1-4GlcNAcβ1)$_n$-3Galβ1-4GlcNAc
NeuAcα2-eGalβ1-4GlcNAcβ1(-3Galβ1-4Galβ1)$_n$-3Galp1-4GlcNAc

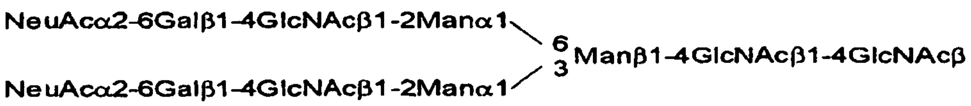

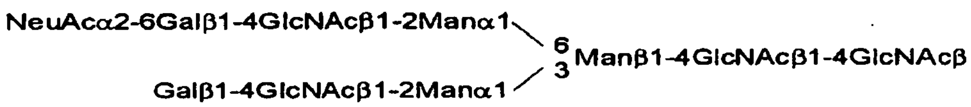

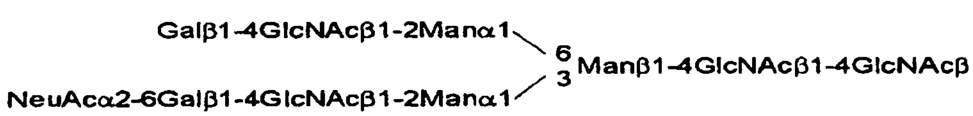

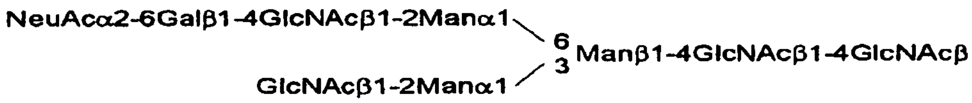

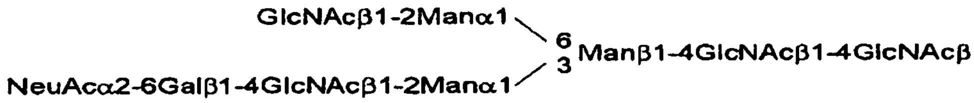

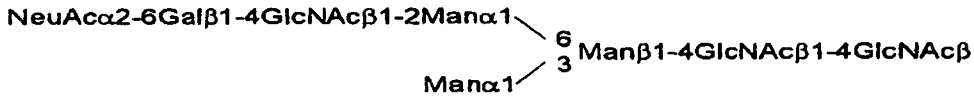

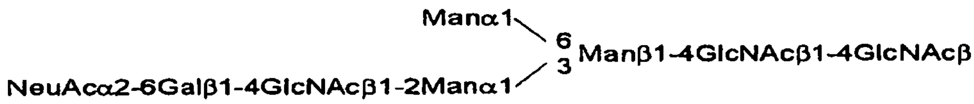

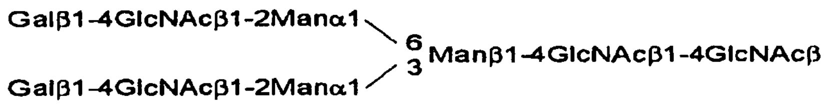

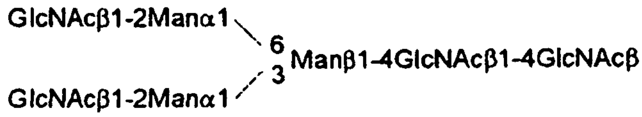

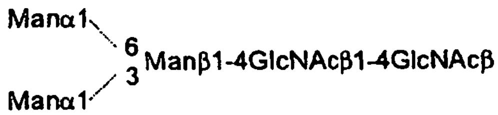

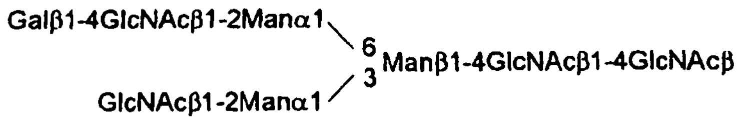

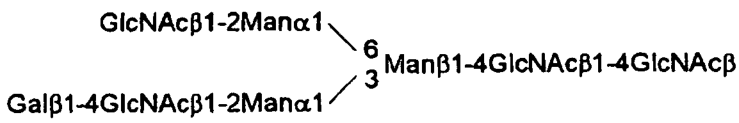

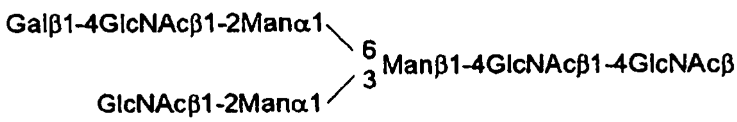

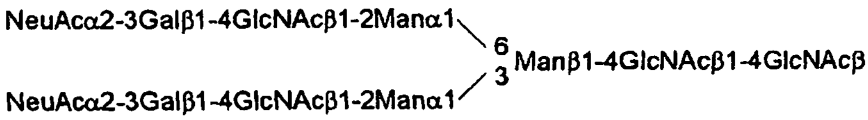

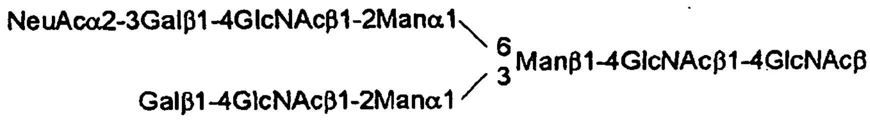

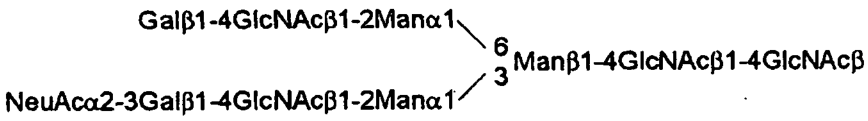

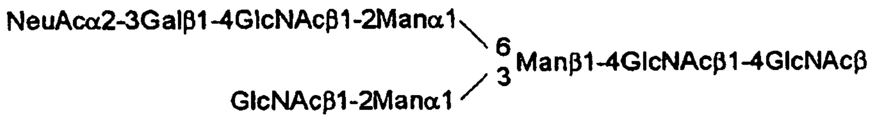

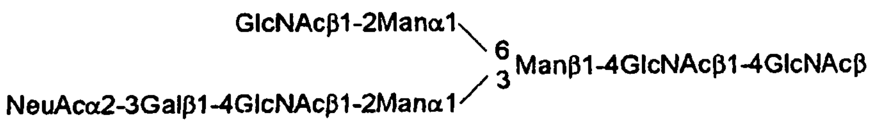

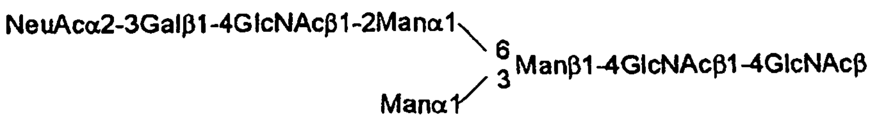

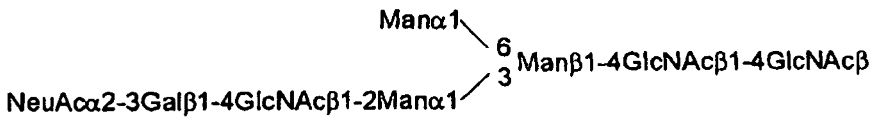

NeuAcα2-6(NeuAca2-3)Galβ1-4GlcNAc and Gen,
Xvv is Asn or a glycosylated amino acid residue selected from the group A of glycosylated amino acid residues,
Ycc is Ala or a glycosylated amino acid residue selected from the group A of glycosylated amino acid residues,
46-position is absent or a glycosylated amino acid residue selected from the group A of glycosylated amino acid residues,
wherein one to four residue(s) of Xqq, Xvv, Ycc and/or 46-position is (are) a glycosylated amino acid residue selected from the group A of glycosylated amino acid residues,
or its ester, amide, alkylamide or dialkylamide; and/or a pharmaceutically acceptable salt thereof.

**3.** A pharmaceutical composition comprising the glycosylated GLP-1 related peptide of claim 1 or 2 as an effective ingredient.

**4.** Medicine for treating or preventing diabetes comprising the glycosylated GLP-1 related peptide of claim 1 or 2 as an effective ingredient.

## Patentansprüche

1. Glycosyliertes GLP-1-verwandtes Peptidderivat der Formel:

| | | | | | | | | | | |
|---|---|---|---|---|---|---|---|---|---|---|
| 7 | 8 | 9 | 10 | 11 | 12 | 13 | 14 | 15 | 16 | 17 |
| His | Ala | Glu | Gly | Thr | Phe | Thr | Ser | Asp | Val | Ser |
| 18 | 19 | 20 | 21 | 22 | 23 | 24 | 25 | 26 | 27 | 28 |
| Ser | Tyr | Leu | Glu | Gly | Gln | Ala | Ala | Xpp | Glu | Phe |
| 29 | 30 | 31 | 32 | 33 | 34 | 35 | 36 | 37 | | |
| Ile | Ala | Trp | Leu | Val | Xvv | Gly | Arg | Xyy | | |

wobei Xpp Lys oder ein glycosylierter Aminosäurerest ist, der aus der Gruppe A glykosylierte Aminosäurereste enthaltend

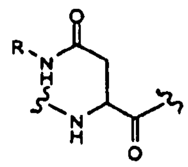
(c) ,

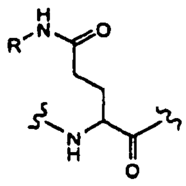
(d) ,

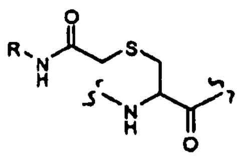
(g) ,

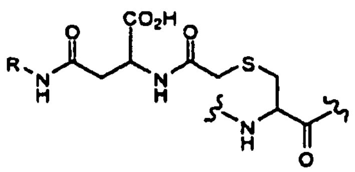
(g') .

oder

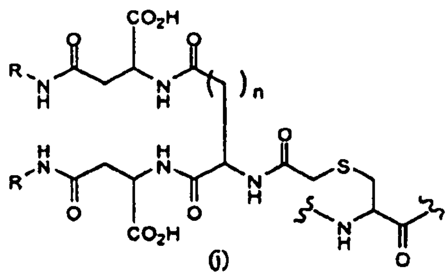
(j)

ausgewählt wird, wobei n eine ganze Zahl von 1 bis 10 ist, und wobei R die Glycokette ist, ausgewählt aus
GlcNAc
Galβ1-4GlcNAc
NeuAcα2-6Galβ1-4GlcNAc
Galβ1-4(Fucα1-3)GlcNAc
NeuAcα2-3Galβ1-4GlcNAc

NeuAcαL2-3Galβ1-4(Fucα1-3)GlcNAc
NeuAcα2-6Galβ1-4(Fucα1-3)GlcNAc
GlcNAcβ1(-3Galβ1-4GlcNAcβ1)$_n$-3Galβ1-4GlcNAc
Galβ1-4GlcNAcβ1 (-3Galβ1-4GlcNAcβ1)$_n$-3Galβ1-4GlcNAc
NeuAcα2-3Galβ1-4GlcNAcβ1(-3Galβ1-4GlcNAcβ1)$_n$-3Galβ1-4GlcNAc
NeuAcα2-6Galβ1-4GlcNAcβ1 (-3Galβ1-4GlcNAcβ1)$_n$-3Galβ1-4GlcNAc

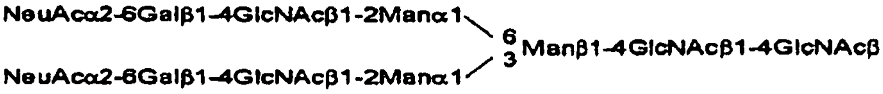

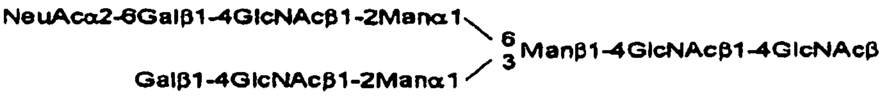

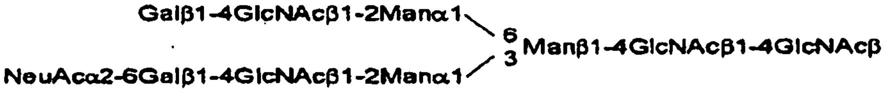

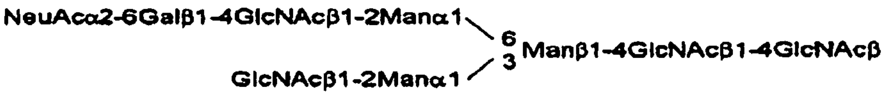

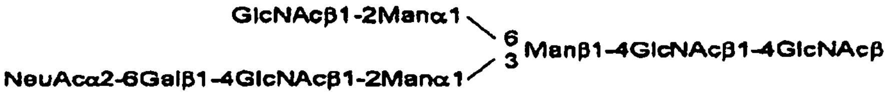

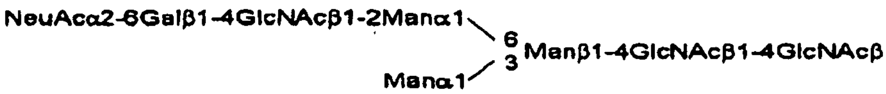

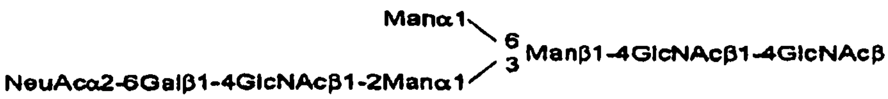

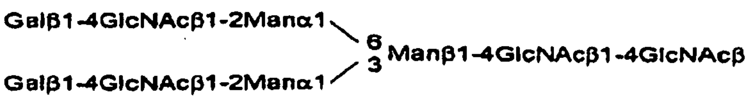

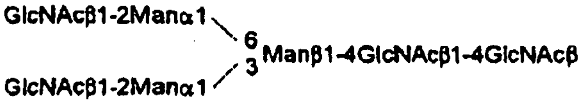

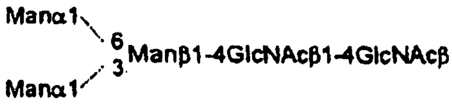

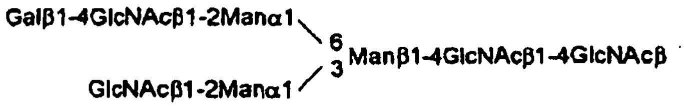

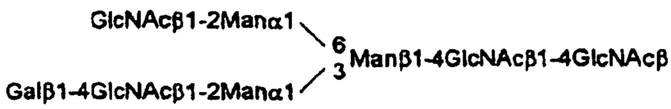

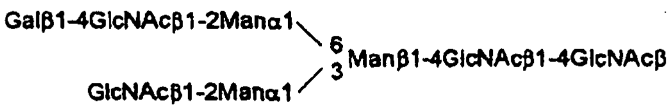

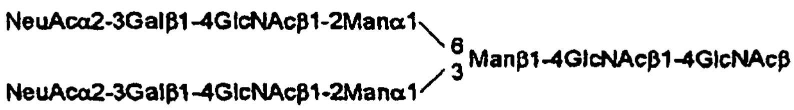

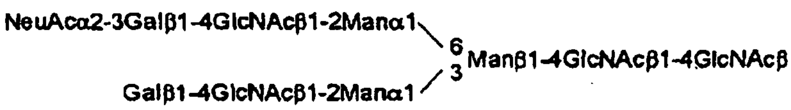

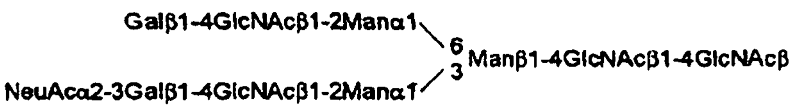

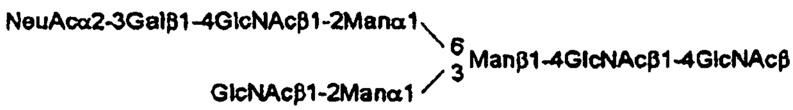

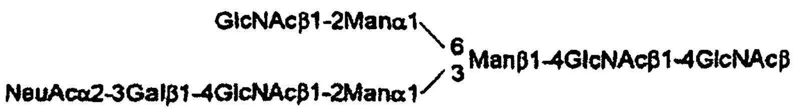

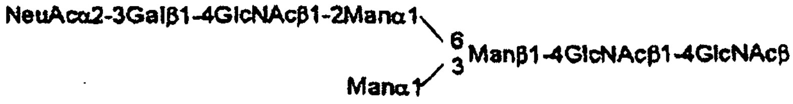

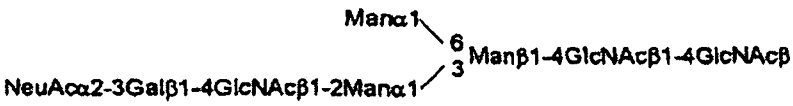

NeuAcα2-6(NeuAcα2-3)Galβ1-4GlcNAc und Gen,

Xvv Lys oder ein glycosylierter Aminosäurerest, ausgewählt aus der Gruppe A von glycosylierten Aminosäureresten ist,

Xyy Gly oder ein glycosylierter Aminosäurerest, ausgewählt aus der Gruppe A von glycosylierten Aminosäureresten ist,

wobei ein bis drei Rest(e) von Xpp, Xvv und/oder Xyy ein glycosylierter Aminosäurerest ist/sind, ausgewählt aus der Gruppe A von glycosylierten Aminosäureresten, oder sein Ester, Amid, Alkylamid oder Dialkylamid; und/oder ein pharmazeutisch annehmbares Salz davon.

**2.** Glycosyliertes GLP-1-verwandtes Peptidderivat der Formel unten:

| | | | | | | | | | | |
|---|---|---|---|---|---|---|---|---|---|---|
| 7 | 8 | 9 | 10 | 11 | 12 | 13 | 14 | 15 | 16 | 17 |
| His | Gly | Glu | Gly | Thr | Phe | Thr | Ser | Asp | Leu | Ser |
| 18 | 19 | 20 | 21 | 22 | 23 | 24 | 25 | 26 | 27 | 28 |
| Lys | Gln | Met | Glu | Glu | Glu | Ala | Val | Arg | Xqq | Phe |
| 29 | 30 | 31 | 32 | 33 | 34 | 35 | 36 | 37 | 38 | 39 |
| Ile | Glu | Trp | Leu | Lys | Xvv | Gly | Gly | Pro | Ser | Ser |
| 40 | 41 | 42 | 43 | 44 | 45 | | | | | |
| Gly | Ycc | Pro | Pro | Pro | Ser | | | | | |

wobei Xqq Leu oder ein glycosylierter Aminosäurerest ist, der aus der Gruppe A von glycosylierten Aminosäureresten, enthaltend

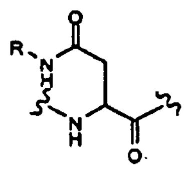

(c)

,

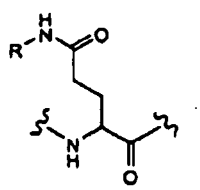

(d)

,

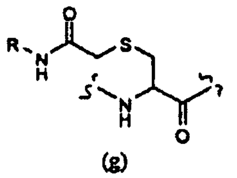

(g) ,

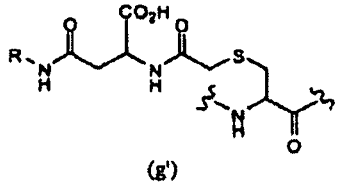

(g') .

oder

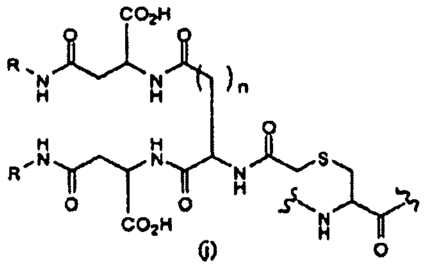

(j)

ausgewählt ist, wobei n eine ganze Zahl von 1 bis 10 ist, und R die Glycokette ist, ausgewählt aus
GlcNAc
Galβ1-4GlcNAc
NeuAcα2-6Galβ1-4GlcNAC
Galβ1-4(Fucα1-3)GlcNAc
NeuAcα2-3Galβ1-4GlcNAc
NeuAcα2-3Galβ1-4(Fucα1-3)GlcNAc
NeuAcα2-6Galβ1-4(Fucα1-3)GlcNAc
GlcNAcβ1(-3Galβ1-4GlcNAcβ1)$_n$-3Galβ1-4GlcNAc

Gelβ1-4GlcNAcβ1(-3Galβ1-AGlaNAcβ1)$_n$-3Galβ1-4GlcNAc
NeuAcα2-3Galβ1-4GlcNAcβ1(-3Galβ1-4GlcNAcβ1)$_n$-3Galβ1-4GalNAc
NeuAcα2-6Galβ1-4GlcNAcβ1(-3Galβ1-4GlcNAcβ1)$_n$-3Galβ1-4GlcNAc

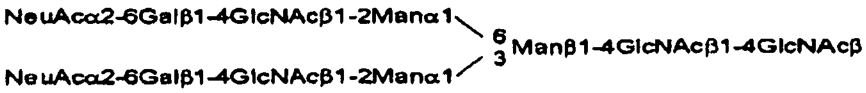

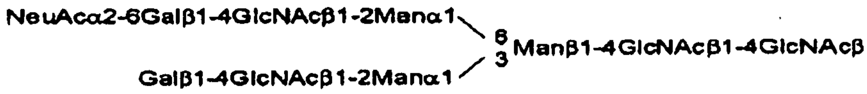

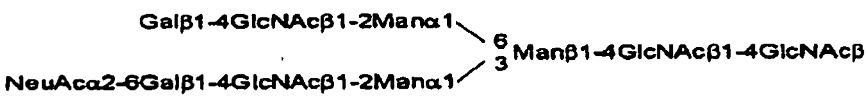

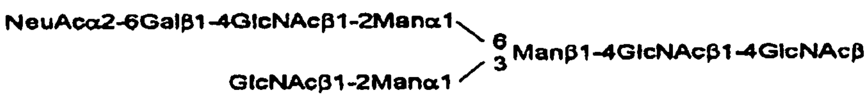

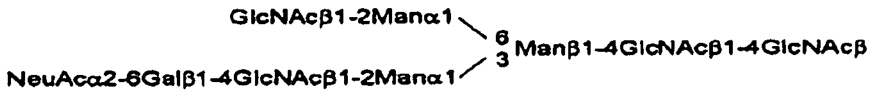

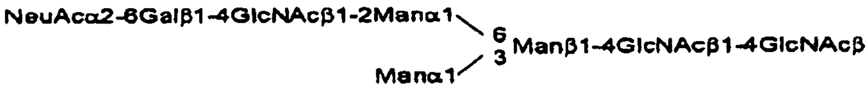

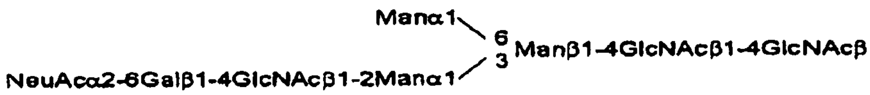

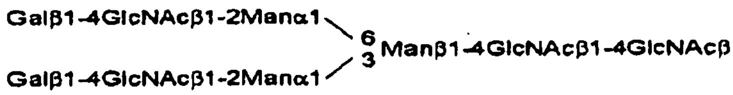

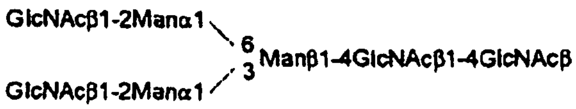

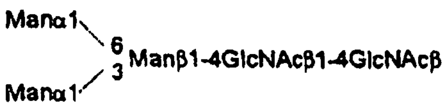

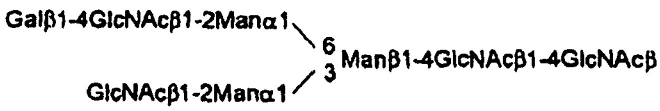

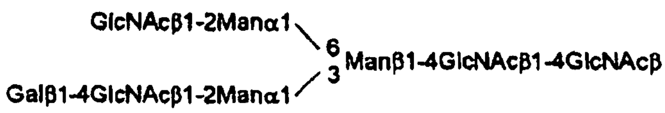

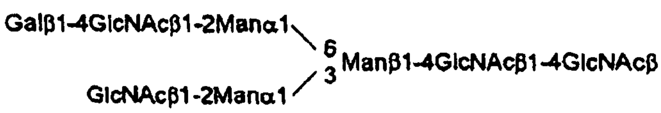

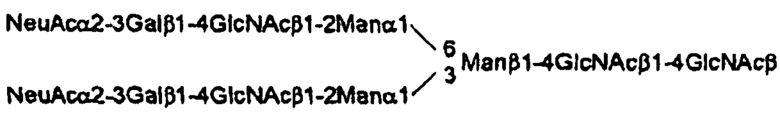

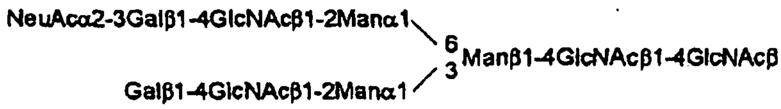

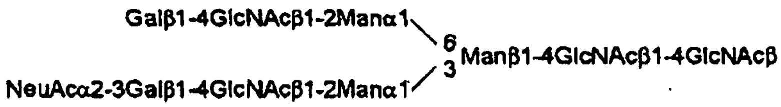

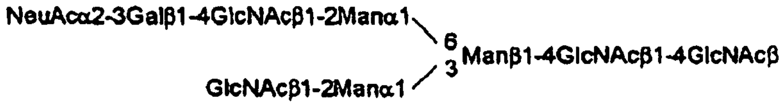

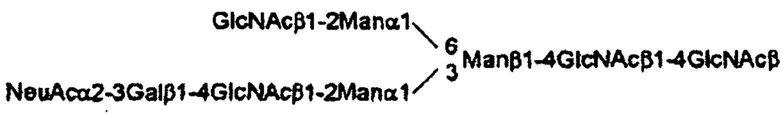

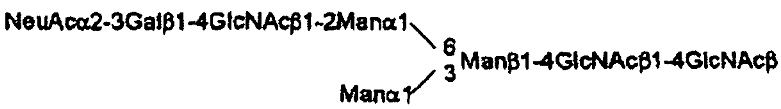

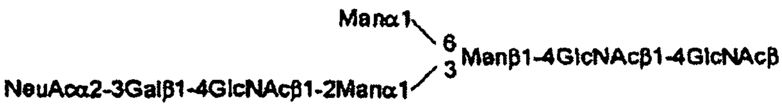

NeuAcαβ-6(NeuAca2-3)Galβ1-4GlcNAc und Gen,
Xvv Asn oder ein glycosylierter Aminosäurerest, ausgewählt aus der Gruppe A von glycosylierten Aminosäureresten ist,
Ycc Ala oder ein glycosylierter Aminosäurerest, ausgewählt aus der Gruppe A von glycosylierten Aminosäureresten ist,
die 46-Position abwesend ist oder ein glycosylierter Aminosäurerest, ausgewählt aus der Gruppe A von glycosylierten Aminosäureresten ist,
wobei ein bis vier Rest(e) von Xqq, Xvv, Ycc und/oder die 46-Position ein glycosylierter Aminosäurerest ist/sind, ausgewählt aus der Gruppe A von glycosylierten Aminosäureresten,
oder sein Ester, Amid, Alkylamid oder Dialkylamid; und/oder ein pharmazeutisch annehmbares Salz davon.

3. Pharmazeutische Zusammensetzung, umfassend das glycosylierte GLP-1-verwandte Peptid von Anspruch 1 oder 2 als wirksamen Bestandteil.

4. Arznei zum Behandeln oder Vorbeugen von Diabetes, umfassend das glycosylierte GLP-1-verwandte Peptid von Anspruch 1 oder 2 als wirksamen Bestandteil.

## Revendications

1. Dérivé de peptide apparenté à GLP-1 glycosylé de formule :

| | | | | | | | | | | |
|---|---|---|---|---|---|---|---|---|---|---|
| 7 | 8 | 9 | 10 | 11 | 12 | 13 | 14 | 15 | 16 | 17 |
| His | Ala | Glu | Gly | Thr | Phe | Thr | Ser | Asp | Val | Ser |
| 18 | 19 | 20 | 21 | 22 | 23 | 24 | 25 | 26 | 27 | 28 |
| Ser | Tyr | Leu | Glu | Gly | Gln | Ala | Ala | Xpp | Glu | Phe |
| 29 | 30 | 31 | 32 | 33 | 34 | 35 | 36 | 37 | | |
| Ile | Ala | Trp | Leu | Val | Xvv | Gly | Arg | Xyy | | |

où Xpp est Lys ou un résidu d'aminoacide glycosylé choisi dans le groupe A de résidus d'aminoacides glycosylés contenant

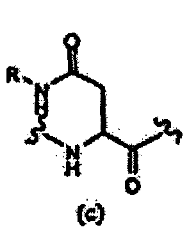
(c) ,

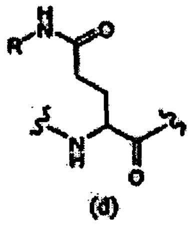
(d) ,

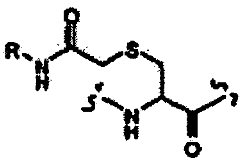
(g) ,

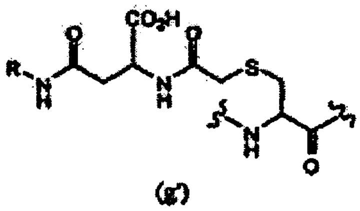
(g') ,

ou

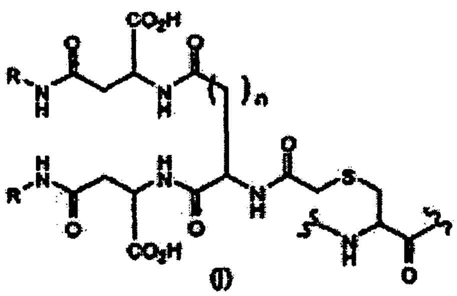
(l)

où n est un entier de 1 à 10 et R est la glycochaîne choisie parmi
GlcNAc
Galβ1-4GlcNAc
NeuAcα2-6Galβ1-4GlcNAc
Galβ1-4(Fucα1-3)FlcNAc
NeuAcα2-3Galβ1-4GlcNAc
NeuAcα2-3Galβ1-4(Fucα1-3)GlcNAc
NeuAcα2-6Galβ1-4(Fucα1-3)GlcNAc

GlcNAcβ1(-3Galβ1-4GlcNAcβ1)$_n$-3Galβ1-4GlcNAc
Galβ1-4GlcNacβ1(-3Galβ1-4GlcNacβ1)$_n$-3Galβ1-4GlcNAc

NeuAcα2-3Galβ1-4GlcNAcβ1(-3Galβ1-4GlcNacβ1)$_n$-3Galβ1-4GlcNAc
NeuAcα2-6Galβ1-4GlcNAcβ1(-3Galβ1-4GlcNacβ1)$_n$-3Galβ1-4GlcNAc

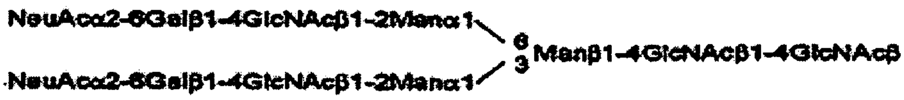

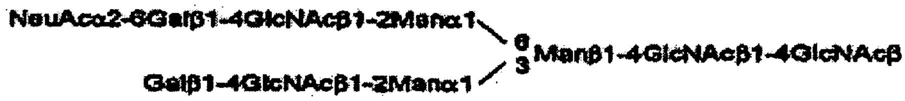

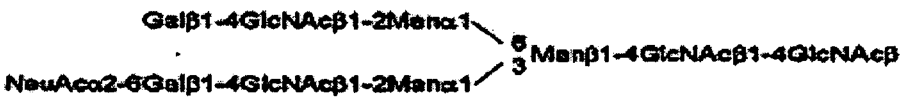

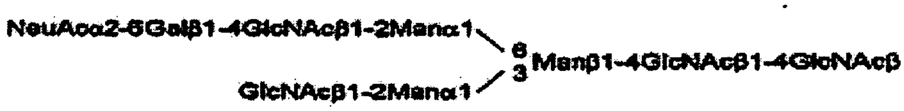

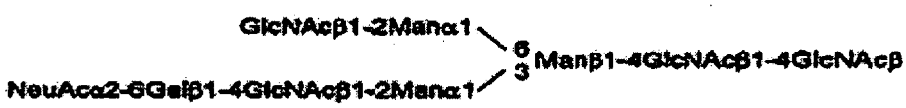

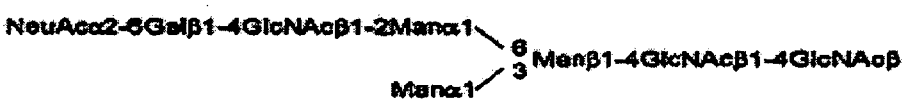

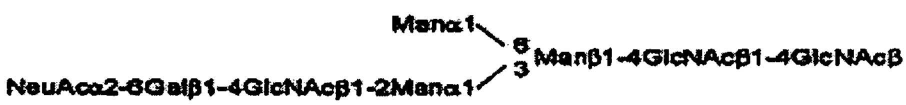

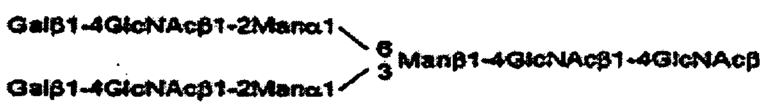

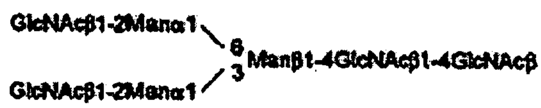

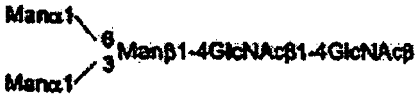

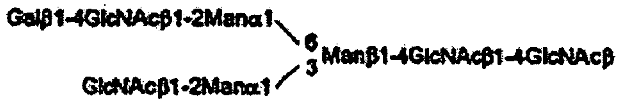

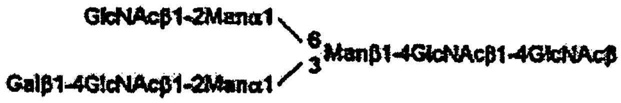

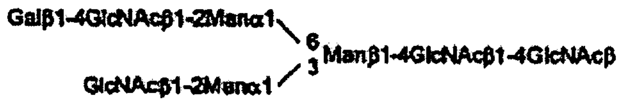

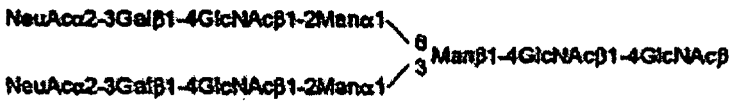

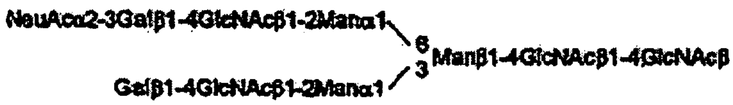

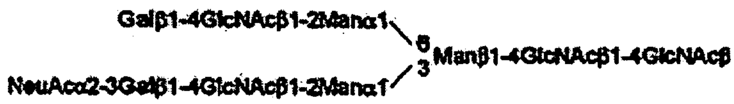

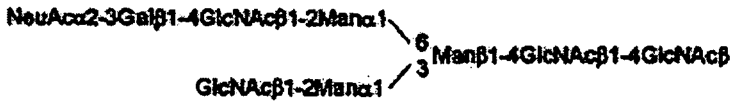

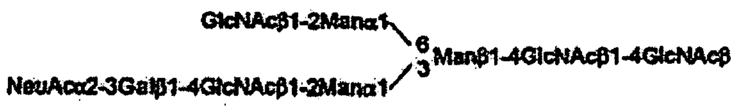

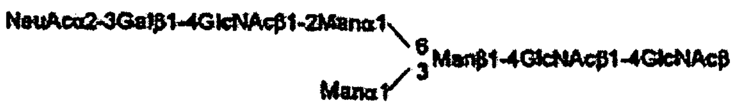

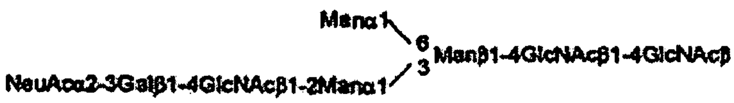

euAcα2-6(NeuAca2-3)Galβ1-4GlcNAc et Gen,
Xvv est Lys ou un résidu d'aminoacide glycosylé choisi dans le groupe A de résidus d'aminoacides glycosylés,
Xyy est Gly ou un résidu d'aminoacide glycosylé choisi dans le groupe A de résidus d'aminoacides glycosylés,
où un à trois résidus de Xpp, Xw et/ou Xyy est (sont) un résidu d'aminoacide glycosylé choisi dans le groupe A de résidus d'aminoacides glycosylés,
où son ester, amide, alkylamide ou dialkylamide ; et/ou sel pharmaceutiquement acceptable de celui-ci.

**2.** Dérivé de peptide apparenté à GLP-1 glycosylé de formule ci-dessous ;

| | | | | | | | | | | |
|---|---|---|---|---|---|---|---|---|---|---|
| 7 | 8 | 9 | 10 | 11 | 12 | 13 | 14 | 15 | 16 | 17 |
| His | Gly | Glu | Gly | Thr | Phe | Thr | Ser | Asp | Leu | Ser |
| 18 | 19 | 20 | 21 | 22 | 23 | 24 | 25 | 26 | 27 | 28 |
| Lys | Gln | Met | Glu | Glu | Glu | Ala | Val | Arg | Xqq | Phe |
| 29 | 30 | 31 | 32 | 33 | 34 | 35 | 36 | 37 | 38 | 39 |
| Ile | Glu | Trp | Leu | Lys | Xvv | Gly | Gly | Pro | Ser | Ser |
| 40 | 41 | 42 | 43 | 44 | 45 | | | | | |
| Gly | Ycc | Pro | Pro | Pro | Ser | | | | | |

où Xqq est Leu ou un résidu d'aminoacide glycosylé choisi dans le groupe A de résidus d'aminoacides glycosylés contenant

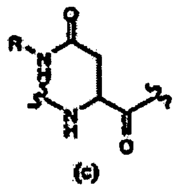
(c) ,
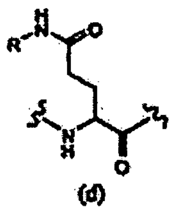
(d) ,

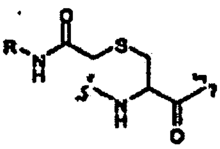
(g) ,
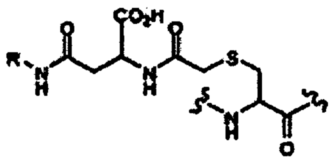
(g') ,

ou

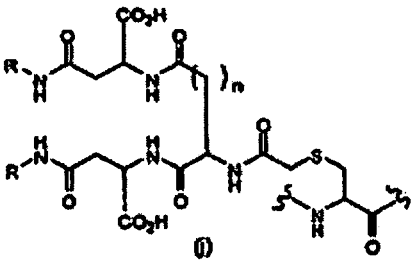
(f)

où n est un entier de 1 à 10 et R est la glycochaîne choisie parmi
GlcNAc
Galβ1-4GlcNAc
NeuAcα2-6Galβ1-4GlcNAc
Galβ1-4(Fucα1-3)GlcNAc
NeuAcα2-3Galβ1-4GlcNAc
NeuAcα2-3Galβ1-4(Fucα1-3)GlcNAc
NeuAcα2-6Galβ1-4(Fucα1-3)GlcNAc
GlcNAcβ1(-3Galβ1-4GlcNacβ1)$_n$-3Galβ1-4GlcNAc
Galβ1-4GlcNAcβ1(-3Galβ1-4GlcNAcβ1)$_n$-3Galβ1-4GlcNAc
NeuAcα2-3Galβ1-4GlcNAcβ1(-3Galβ1-4GlcNAcβ1)$_n$-3Galβ1-4GlcNAc
NeuAcα2-6Galβ1-4GlcNAcβ1(-3Galβ1-4GlcNAcβ1)$_n$-3Galβ1-4GlcNAc

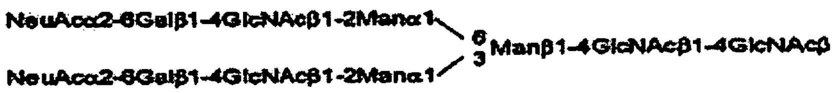

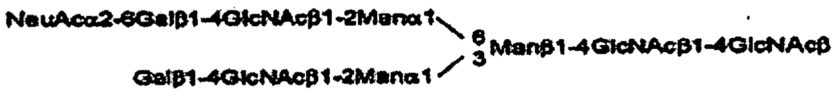

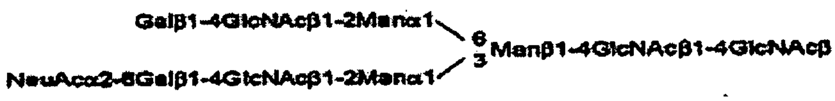

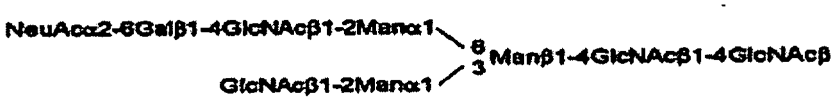

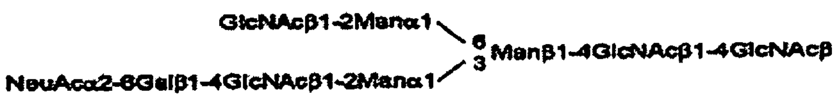

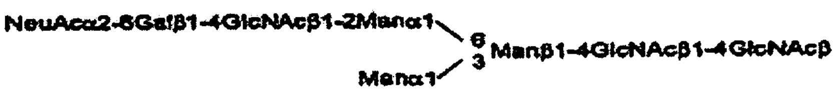

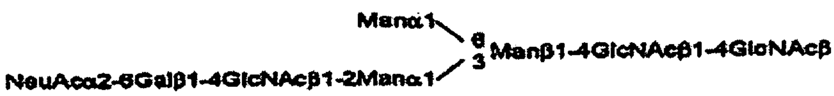

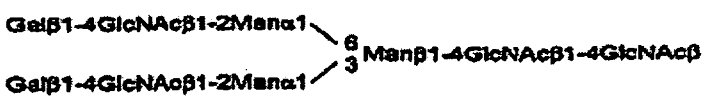

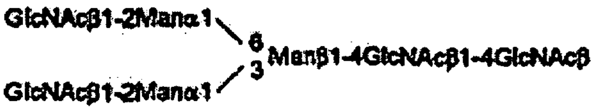

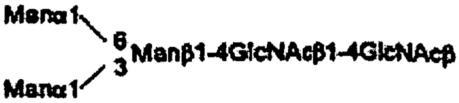

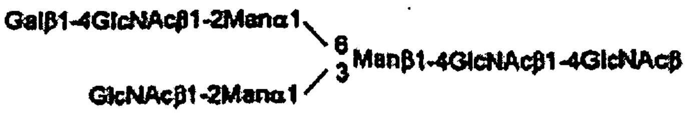

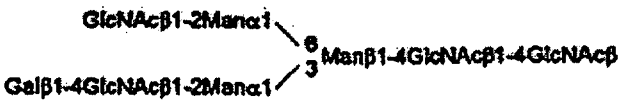

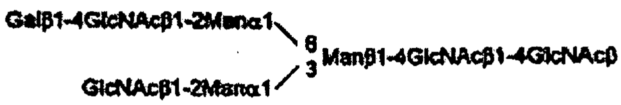

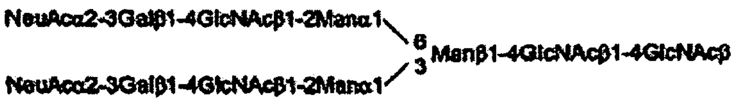

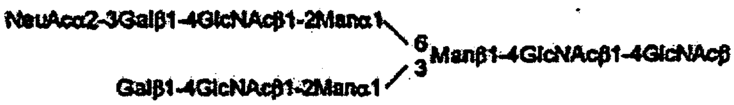

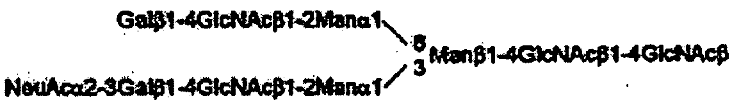

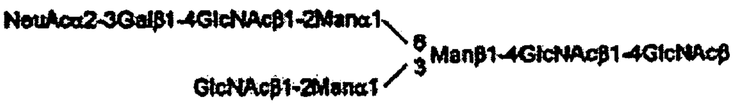

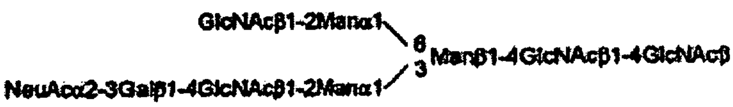

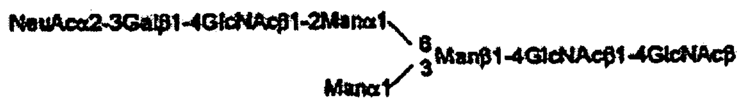

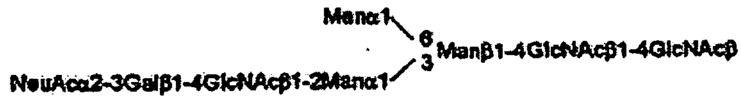

NeuAcα2-6(NeuAcα2-3)Galβ1-4GlcNAc et Gen,
Xvv est Asn ou un résidu d'aminoacide glycosylé choisi dans le groupe A de résidus d'aminoacides glycosylés,
Ycc est Ala ou un résidu d'aminoacide glycosylé choisi dans le groupe A de résidus d'aminoacides glycosylés,
la position 46 est absente ou un résidu d'aminoacide glycosylé choisi dans le groupe A de résidus d'aminoacides glycosylés,
où un à quatre résidus de Xqq, Xvv, Ycc et/ou la position 46 est (sont) un résidu d'aminoacide glycosylé choisi dans le groupe A de résidus d'aminoacides glycosylés,
ou son ester, amide, alkylamide ou dialkylamide ; et/ou sel pharmaceutiquement acceptable de celui-ci.

**3.** Composition pharmaceutique comprenant le peptide apparenté à GLP-1 glycosylé selon la revendication 1 ou 2 comme ingrédient efficace.

**4.** Médicament pour traiter ou prévenir le diabète comprenant le peptide apparenté à GLP-1 glycosylé selon la revendication 1 ou 2 comme ingrédient efficace.

Fig. 1

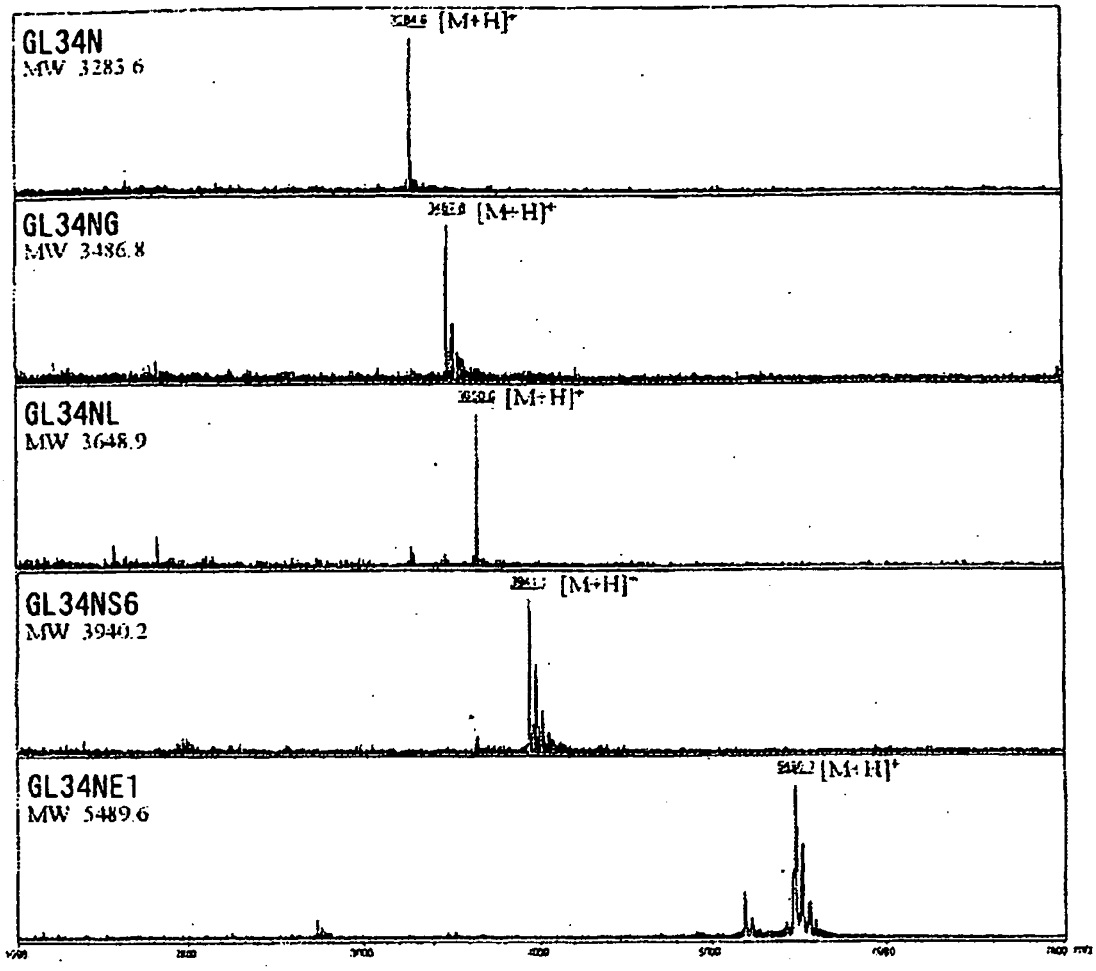
GL34N
MW 3283.6
[M+H]+
GL34NG
MW 3486.8
[M+H]+
GL34NL
MW 3648.9
[M+H]+
GL34NS6
MW 3940.2
[M+H]+
GL34NE1
MW 5489.6
[M+H]+

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 272097 A
- WO 9318785 A
- JP 06833637 B
- JP 2005346905 A


### Non-patent literature cited in the description


- *Lancet,* 1987, vol. 2, 1300-4
- *Regul Pept,* 2005, vol. 128, 135-48
- *Eur J Biochem,* 1993, vol. 214, 829-35
- *J Biol Chem,* 1997, vol. 272, 21201-6
- *Diabetologia,* 1998, vol. 41, 271-278
- *Regul Pept,* 2001, vol. 96, 95-104
- *Regul Pept,* 2000, vol. 86, 103-111
- *Regul Pept,* 1999, vol. 79, 93-102
- *JBC,* 2004, vol. 279, 3998-4006
- *J Endocrinol,* 1998, vol. 159, 93-102
- *Metabolism,* 1999, vol. 48, 252-258
- *Biol Chem,* 2003, vol. 384, 1543-1551
- Remington: Pharmacentical Science. 1985
- Remington: The Science and Practice of Pharmacy. 1995

SEQUENCE LISTING

<110> SHIONOGI & CO., LTD.; NATIONAL UNIVERSITY CORPORATION HOKKAIDO UNIVERSITY
<120> GLYCOSYLATED PEPTIDES AND MEDICINE COMPRISING IT AS AN EFFECTIVE INGREDIENT

<130> P99160EP00

<140> 06833637.9
<141> 2006-11-29

<150> JP2005-346905
<151> 2005-11-30

<160> 59

<210> 1
<211> 30
<212> PRT
<213> Artificial Sequence

<220>
<221> AMIDATION
<222> 36
<223> Glucagon-Like Peptide(7-36)

<400> 1

His Ala Glu Gly Thr Phe Thr Ser Asp Val
7 10 15

Ser Ser Tyr Leu Glu Gly Gln Ala Ala Lys Glu Phe Ile Ala Trp Leu
20 25 30

Val Lys Gly Arg
35

<210> 2
<211> 30
<212> PRT
<213> Artificial Sequence

<220>
<221> AMIDATION
<222> 36
<223> Modified GL(7-36)

<220>
<221>CARBOHYD
<222>8
<223>N-LINKED

<400> 2

His Asn Glu Gly Thr Phe Thr Ser Asp Val
7 10 15

Ser Ser Tyr Leu Glu Gly Gln Ala Ala Lys Glu Phe Ile Ala Trp Leu
20 25 30

Val Lys Gly Arg
35

<210> 3
<211> 30
<212> PRT
<213> Artificial Sequence

<220>
<221> AMIDATION
<222> 36
<223> Modified GLP(7-36)

<220>
<221>CARBOHYD
<222>19
<223>N-LINKED

<400> 3

His Ala Glu Gly Thr Phe Thr Ser Asp Val
7 10 15

Ser Ser Asn Leu Glu Gly Gln Ala Ala Lys Glu Phe Ile Ala Trp Leu
20 25 30

Val Lys Gly Arg
35

<210> 4
<211> 30
<212> PRT
<213> Artificial Sequence

<220>
<221> AMIDATION
<222> 36
<223> Modified GLP(7-36)

<220>
<221>CARBOHYD
<222>26
<223>N-LINKED

<400> 4

His Ala Glu Gly Thr Phe Thr Ser Asp Val
7 10 15

Ser Ser Tyr Leu Glu Gly Gln Ala Ala Asn Glu Phe Ile Ala Trp Leu
20 25 30

Val Lys Gly Arg
35

<210> 5
<211> 30
<212> PRT
<213> Artificial Sequence

<220>
<221> AMIDATION
<222> 36
<223> Modified GLP(7-36)

<220>
<221>CARBOHYD
<222>34
<223>N-LINKED

<400> 5

His Ala Glu Gly Thr Phe Thr Ser Asp Val
7 10 15

Ser Ser Tyr Leu Glu Gly Gln Ala Ala Lys Glu Phe Ile Ala Trp Leu
20 25 30

Val Asn Gly Arg
35

<210> 6
<211> 31
<212> PRT
<213> Artificial Sequence

<220>
<221> AMIDATION
<222> 37
<223> Modified GLP(7-36)

<220>
<221>CARBOHYD
<222>37
<223>N-LINKED

<400> 6

His Ala Glu Gly Thr Phe Thr Ser Asp Val
7 10 15

Ser Ser Tyr Leu Glu Gly Gln Ala Ala Lys Glu Phe Ile Ala Trp Leu
20 25 30

Val Lys Gly Arg Asn
35

<210> 7
<211> 37
<212> PRT
<213> Artificial Sequence

<220>
<221> AMIDATION
<222> 43
<223> Modified GLP(7-36)

<220>
<221>CARBOHYD
<222>43
<223>N-LINKED

<400> 7

His Ala Glu Gly Thr Phe Thr Ser Asp Val
7 10 15

Ser Ser Tyr Leu Glu Gly Gln Ala Ala Lys Glu Phe Ile Ala Trp Leu
20 25 30

Val Lys Gly Arg Ser Gly Ser Gly Ser Gly Asp
35 40

<210> 8
<211> 30
<212> PRT
<213> Artificial Sequence

<220>
<221> AMIDATION
<222> 36
<223> Modified GLP(7-36)

<220>
<221>CARBOHYD
<222>26
<223>N-LINKED

<220>
<221>CARBOHYD
<222>34
<223>N-LINKED

<400> 8

His Ala Glu Gly Thr Phe Thr Ser Asp Val
7 10 15

Ser Ser Tyr Leu Glu Gly Gln Ala Ala Asn Glu Phe Ile Ala Trp Leu
20 25 30

Val Asn Gly Arg
35

<210> 9
<211> 31
<212> PRT
<213> Artificial Sequence

<220>
<221> AMIDATION
<222> 37
<223> Modified GLP(7-36)

<220>
<221>CARBOHYD
<222>26
<223>N-LINKED

<220>
<221>CARBOHYD
<222>37

<223>N-LINKED

<400> 9

His Ala Glu Gly Thr Phe Thr Ser Asp Val
7 10 15

Ser Ser Tyr Leu Glu Gly Gln Ala Ala Asn Glu Phe Ile Ala Trp Leu
20 25 30

Val Lys Gly Arg Asn
35

<210> 10
<211> 31
<212> PRT
<213> Artificial Sequence

<220>
<221> AMIDATION
<222> 37
<223> Modified GLP(7-36)

<220>
<221>CARBOHYD
<222>34
<223>N-LINKED

<220>
<221>CARBOHYD
<222>37
<223>N-LINKED

<400> 10

His Ala Glu Gly Thr Phe Thr Ser Asp Val
7 10 15

Ser Ser Tyr Leu Glu Gly Gln Ala Ala Lys Glu Phe Ile Ala Trp Leu
20 25 30

Val Asn Gly Arg Asn
35

<210> 11
<211> 31
<212> PRT
<213> Artificial Sequence

<220>
<221> AMIDATION
<222> 37
<223> Modified GLP(7-36)

<220>
<221> CARBOHYD
<222> 34

<223> N-LINKED

<220>
<221> CARBOHYD
<222> 37
<223> N-LINKED

<400> 11

His Ala Glu Gly Thr Phe Thr Ser Asp Val
7 10 15

Ser Ser Tyr Leu Glu Gly Gln Ala Ala Lys Glu Phe Ile Ala Trp Leu
20 25 30

Val Cys Gly Arg Cys
35

<210> 12
<211> 31
<212> PRT
<213> Artificial Sequence

<220>
<221> AMIDATION
<222> 37
<223> Modified GLP(7-36)

<220>
<221>CARBOHYD
<222>26
<223>N-LINKED

<220>
<221>CARBOHYD
<222>34
<223>N-LINKED

<220>
<221>CARBOHYD
<222>37
<223>N-LINKED

<400> 12

His Ala Glu Gly Thr Phe Thr Ser Asp Val
7 10 15

Ser Ser Tyr Leu Glu Gly Gln Ala Ala Asn Glu Phe Ile Ala Trp Leu
20 25 30

Val Asn Gly Arg Asn
35

<210> 13
<211> 39
<212> PRT
<213> Artificial Sequence

<220>
<221> AMIDATION
<222> 39
<223> Exendin-4

<400> 13

```
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35
```

<210> 14
<211> 40
<212> PRT
<213> Artificial Sequence

<220>
<221> AMIDATION
<222> 39
<223> Modified Exendin-4

<220>
<221> CARBOHYD
<222> 0
<223> N-LINKED

<400> 14

```
Asn His Gly Glu Gly Thr Phe Thr Ser Asp Leu Ser Lys Gln Met Glu
    1               5                   10                  15

Glu Glu Ala Val Arg Leu Phe Ile Glu Trp Leu Lys Asn Gly Gly Pro
                20                  25                  30

Ser Ser Ser Gly Ala Pro Pro Pro Ser
            35
```

<210> 15
<211> 39
<212> PRT
<213> Artificial Sequence

<220>
<221> AMIDATION
<222> 39
<223> Modified Exendin-4

<220>
<221> CARBOHYD
<222> 1
<223> N-LINKED

<400> 15

Asn Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Ser Gly Ala Pro Pro Pro Ser
35

<210> 16
<211> 39
<212> PRT
<213> Artificial Sequence

<220>
<221> AMIDATION
<222> 39
<223> Modified Exendin-4

<220>
<221> CARBOHYD
<222> 2
<223> N-LINKED

<400> 16

His Asn Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Ser Gly Ala Pro Pro Pro Ser
35

<210> 17
<211> 39
<212> PRT
<213> Artificial Sequence

<220>
<221> AMIDATION
<222> 39
<223> Modified Exendin-4

<220>
<221> CARBOHYD
<222> 3
<223> N-LINKED

<400> 17

```
His Gly Asn Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35
```

<210> 18
<211> 39
<212> PRT
<213> Artificial Sequence

<220>
<221> AMIDATION
<222> 39
<223> Modified Exendin-4

<220>
<221> CARBOHYD
<222> 4
<223> N-LINKED

<400> 18

```
His Gly Glu Asn Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35
```

<210> 19
<211> 39
<212> PRT
<213> Artificial Sequence

<220>
<221> AMIDATION
<222> 39
<223> Modified Exendin-4

<220>
<221> CARBOHYD
<222> 5
<223> N-LINKED

<400> 19

```
His Gly Glu Gly Asn Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35
```

<210> 20
<211> 39
<212> PRT
<213> Artificial Sequence

<220>
<221> AMIDATION
<222> 39
<223> Modified Exendin-4

<220>
<221> CARBOHYD
<222> 6
<223> N-LINKED

<400> 20

His Gly Glu Gly Thr Asn Thr Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Ser Gly Ala Pro Pro Pro Ser
35

<210> 21
<211> 39
<212> PRT
<213> Artificial Sequence

<220>
<221> AMIDATION
<222> 39
<223> Modified Exendin-4

<220>
<221> CARBOHYD
<222> 7
<223> N-LINKED

<400> 21

His Gly Glu Gly Thr Phe Asn Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Ser Gly Ala Pro Pro Pro Ser
35

<210> 22
<211> 39
<212> PRT
<213> Artificial Sequence

<220>
<221> AMIDATION

<222> 39
<223> Modified Exendin-4

<220>
<221> CARBOHYD
<222> 8
<223> N-LINKED

<400> 22

His Gly Glu Gly Thr Phe Thr Asn Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210> 23
<211> 39
<212> PRT
<213> Artificial Sequence

<220>
<221> AMIDATION
<222> 39
<223> Modified Exendin-4

<220>
<221> CARBOHYD
<222> 9
<223> N-LINKED

<400> 23

His Gly Glu Gly Thr Phe Thr Ser Asn Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210> 24
<211> 39
<212> PRT
<213> Artificial Sequence

<220>
<221> AMIDATION
<222> 39
<223> Modified Exendin-4

<220>
<221> CARBOHYD
<222> 10
<223> N-LINKED

<400> 24

His Gly Glu Gly Thr Phe Thr Ser Asp Asn Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210> 25
<211> 39
<212> PRT
<213> Artificial Sequence

<220>
<221> AMIDATION
<222> 39
<223> Modified Exendin-4

<220>
<221> CARBOHYD
<222> 11
<223> N-LINKED

<400> 25

His Gly Glu Gly Thr Phe Thr Ser Asp Leu Asn Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210> 26
<211> 39
<212> PRT
<213> Artificial Sequence

<220>
<221> AMIDATION
<222> 39
<223> Modified Exendin-4

<220>
<221> CARBOHYD
<222> 12
<223> N-LINKED

<400> 26

His Gly Glu Gly Thr Phe Thr Ser Asp Leu Ser Asn Gln Met Glu Glu
1               5                   10                  15

Glu Ala Val Arg Leu Phe Ile Glu Trp Leu Lys Asn Gly Gly Pro Ser
20 25 30

Ser Gly Ala Pro Pro Pro Ser
35

<210> 27
<211> 39
<212> PRT
<213> Artificial Sequence

<220>
<221> AMIDATION
<222> 39
<223> Modified Exendin-4

<220>
<221> CARBOHYD
<222> 13
<223> N-LINKED

<400> 27

His Gly Glu Gly Thr Phe Thr Ser Asp Leu Ser Lys Asn Met Glu Glu
1 5 10 15

Glu Ala Val Arg Leu Phe Ile Glu Trp Leu Lys Asn Gly Gly Pro Ser
20 25 30

Ser Gly Ala Pro Pro Pro Ser
35

<210> 28
<211> 39
<212> PRT
<213> Artificial Sequence

<220>
<221> AMIDATION
<222> 39
<223> Modified Exendin-4

<220>
<221> CARBOHYD
<222> 14
<223> N-LINKED

<400> 28

His Gly Glu Gly Thr Phe Thr Ser Asp Leu Ser Lys Gln Asn Glu Glu
1 5 10 15

Glu Ala Val Arg Leu Phe Ile Glu Trp Leu Lys Asn Gly Gly Pro Ser
20 25 30

Ser Gly Ala Pro Pro Pro Ser
35

<210> 29

<211> 39
<212> PRT
<213> Artificial Sequence

<220>
<221> AMIDATION
<222> 39
<223> Modified Exendin-4

<220>
<221> CARBOHYD
<222> 15
<223> N-LINKED

<400> 29

```
His Gly Glu Gly Thr Phe Thr Ser Asp Leu Ser Lys Gln Met Asn Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35
```

<210> 30
<211> 39
<212> PRT
<213> Artificial Sequence

<220>
<221> AMIDATION
<222> 39
<223> Modified Exendin-4

<220>
<221> CARBOHYD
<222> 16
<223> N-LINKED

<400> 30

```
His Gly Glu Gly Thr Phe Thr Ser Asp Leu Ser Lys Gln Met Glu Asn
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35
```

<210> 31
<211> 39
<212> PRT
<213> Artificial Sequence

<220>
<221> AMIDATION

<222> 39
<223> Modified Exendin-4

<220>
<221> CARBOHYD
<222> 17
<223> N-LINKED

<400> 31

```
His Gly Glu Gly Thr Phe Thr Ser Asp Leu Ser Lys Gln Met Glu Glu
1               5                   10                  15

Asn Ala Val Arg Leu Phe Ile Glu Trp Leu Lys Asn Gly Gly Pro Ser
            20                  25                  30

Ser Gly Ala Pro Pro Pro Ser
        35
```

<210> 32
<211> 39
<212> PRT
<213> Artificial sequence

<220>
<221> AMIDATION
<222> 39
<223> Modified Exendin-4

<220>
<221> CARBOHYD
<222> 18
<223> N-LINKED

<400> 32

```
His Gly Glu Gly Thr Phe Thr Ser Asp Leu Ser Lys Gln Met Glu Glu
1               5                   10                  15

Glu Asn Val Arg Leu Phe Ile Glu Trp Leu Lys Asn Gly Gly Pro Ser
            20                  25                  30

Ser Gly Ala Pro Pro Pro Ser
        35
```

<210> 33
<211> 39
<212> PRT
<213> Artificial Sequence

<220>
<221> AMIDATION
<222> 39
<223> Modified Exendin-4

<220>
<221> CARBOHYD
<222> 19
<223> N-LINKED

<400> 33

His Gly Glu Gly Thr Phe Thr Ser Asp Leu Ser Lys Gln Met Glu Glu
1               5                   10                  15

Glu Ala Asn Arg Leu Phe Ile Glu Trp Leu Lys Asn Gly Gly Pro Ser
            20                  25                  30

Ser Gly Ala Pro Pro Pro Ser
        35

<210> 34
<211> 39
<212> PRT
<213> Artificial Sequence

<220>
<221> AMIDATION
<222> 39
<223> Modified Exendin-4

<220>
<221> CARBOHYD
<222> 20
<223> N-LINKED

<400> 34

His Gly Glu Gly Thr Phe Thr Ser Asp Leu Ser Lys Gln Met Glu Glu
1               5                   10                  15

Glu Ala Val Asn Leu Phe Ile Glu Trp Leu Lys Asn Gly Gly Pro Ser
            20                  25                  30

Ser Gly Ala Pro Pro Pro Ser
        35

<210> 35
<211> 39
<212> PRT
<213> Artificial Sequence

<220>
<221> AMIDATION
<222> 39
<223> Modified Exendin-4

<220>
<221> CARBOHYD
<222> 21
<223> N-LINKED

<400> 35

His Gly Glu Gly Thr Phe Thr Ser Asp Leu Ser Lys Gln Met Glu Glu
1               5                   10                  15

Glu Ala Val Arg Asn Phe Ile Glu Trp Leu Lys Asn Gly Gly Pro Ser
            20                  25                  30

Ser Gly Ala Pro Pro Pro Ser
        35

<210> 36
<211> 39
<212> PRT
<213> Artificial Sequence

<220>
<221> AMIDATION
<222> 39
<223> Modified Exendin-4

<220>
<221> CARBOHYD
<222> 22
<223> N-LINKED

<400> 36

His Gly Glu Gly Thr Phe Thr Ser Asp Leu Ser Lys Gln Met Glu Glu
1               5                   10                  15

Glu Ala Val Arg Leu Asn Ile Glu Trp Leu Lys Asn Gly Gly Pro Ser
            20                  25                  30

Ser Gly Ala Pro Pro Pro Ser
        35

<210> 37
<211> 39
<212> PRT
<213> Artificial Sequence

<220>
<221> AMIDATION
<222> 39
<223> Modified Exendin-4

<220>
<221> CARBOHYD
<222> 23
<223> N-LINKED

<400> 37

His Gly Glu Gly Thr Phe Thr Ser Asp Leu Ser Lys Gln Met Glu Glu
1 5 10 15

Glu Ala Val Arg Leu Phe Asn Glu Trp Leu Lys Asn Gly Gly Pro Ser
20 25 30

Ser Gly Ala Pro Pro Pro Ser
35

<210> 38
<211> 39
<212> PRT
<213> Artificial Sequence

<220>
<221> AMIDATION
<222> 39
<223> Modified Exendin-4

<220>
<221> CARBOHYD
<222> 24
<223> N-LINKED

<400> 38

His Gly Glu Gly Thr Phe Thr Ser Asp Leu Ser Lys Gln Met Glu Glu
1 5 10 15

Glu Ala Val Arg Leu Phe Ile Asn Trp Leu Lys Asn Gly Gly Pro Ser
20 25 30

Ser Gly Ala Pro Pro Pro Ser
35

<210> 39
<211> 39
<212> PRT
<213> Artificial Sequence

<220>
<221> AMIDATION
<222> 39
<223> Modified Exendin-4

<220>
<221> CARBOHYD
<222> 25
<223> N-LINKED

<400> 39

His Gly Glu Gly Thr Phe Thr Ser Asp Leu Ser Lys Gln Met Glu Glu
1 5 10 15

Glu Ala Val Arg Leu Phe Ile Glu Asn Leu Lys Asn Gly Gly Pro Ser
20 25 30

Ser Gly Ala Pro Pro Pro Ser
35

<210> 40
<211> 39
<212> PRT
<213> Artificial sequence

<220>
<221> AMIDATION
<222> 39
<223> Modified Exendin-4

<220>
<221> CARBOHYD
<222> 26
<223> N-LINKED

<400> 40

His Gly Glu Gly Thr Phe Thr Ser Asp Leu Ser Lys Gln Met Glu Glu
1 5 10 15

Glu Ala Val Arg Leu Phe Ile Glu Trp Asn Lys Asn Gly Gly Pro Ser
20 25 30

Ser Gly Ala Pro Pro Pro Ser
35

<210> 41
<211> 39
<212> PRT
<213> Artificial sequence

<220>
<221> AMIDATION
<222> 39
<223> Modified Exendin-4

<220>
<221> CARBOHYD
<222> 27
<223> N-LINKED

<400> 41

His Gly Glu Gly Thr Phe Thr Ser Asp Leu Ser Lys Gln Met Glu Glu
1 5 10 15

Glu Ala Val Arg Leu Phe Ile Glu Trp Leu Asn Asn Gly Gly Pro Ser
20 25 30

Ser Gly Ala Pro Pro Pro Ser
35

<210> 42
<211> 39
<212> PRT
<213> Artificial Sequence

<220>
<221> AMIDATION
<222> 39
<223> Modified Exendin-4

<220>
<221> CARBOHYD
<222> 29
<223> N-LINKED

<400> 42

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Asn Gly Pro Ser
20 25 30

Ser Gly Ala Pro Pro Pro Ser
35

<210> 43
<211> 39
<212> PRT
<213> Artificial sequence

<220>
<221> AMIDATION
<222> 39
<223> Modified Exendin-4

<220>
<221> CARBOHYD
<222> 30
<223> N-LINKED

<400> 43

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Asn Pro Ser
20 25 30

Ser Gly Ala Pro Pro Pro Ser
35

<210> 44
<211> 39
<212> PRT
<213> Artificial sequence

<220>
<221> AMIDATION
<222> 39
<223> Modified Exendin-4

<220>
<221> CARBOHYD
<222> 31
<223> N-LINKED

<400> 44

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Asn Ser
20 25 30

Ser Gly Ala Pro Pro Pro Ser
35

<210> 45
<211> 39
<212> PRT
<213> Artificial sequence

<220>
<221> AMIDATION
<222> 39
<223> Modified Exendin-4

<220>
<221> CARBOHYD
<222> 32
<223> N-LINKED

<400> 45

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Pro Asn
20 25 30

Ser Gly Ala Pro Pro Pro Ser
35

<210> 46
<211> 39
<212> PRT
<213> Artificial Sequence

<220>
<221> AMIDATION
<222> 39
<223> Modified Exendin-4

<220>
<221> CARBOHYD
<222> 33
<223> N-LINKED

<400> 46

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Asn Gly Ala Pro Pro Pro Ser
35

<210> 47
<211> 39
<212> PRT
<213> Artificial Sequence

<220>
<221> AMIDATION
<222> 39
<223> Modified Exendin-4

<220>
<221> CARBOHYD
<222> 34
<223> N-LINKED

<400> 47

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Ser Asn Ala Pro Pro Pro Ser
35

<210> 48
<211> 39
<212> PRT
<213> Artificial sequence

<220>
<221> AMIDATION
<222> 39
<223> Modified Exendin-4

<220>
<221> CARBOHYD
<222> 34
<223> N-LINKED

<400> 48

```
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Asn Pro Pro Pro Ser
        35
```

<210> 49
<211> 39
<212> PRT
<213> Artificial Sequence

<220>
<221> AMIDATION
<222> 39
<223> Modified Exendin-4

<220>
<221> CARBOHYD
<222> 36
<223> N-LINKED

<400> 49

```
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Asn Pro Pro Ser
        35
```

<210> 50
<211> 39
<212> PRT
<213> Artificial sequence

<220>
<221> AMIDATION

<222> 39
<223> Modified Exendin-4

<220>
<221> CARBOHYD
<222> 37
<223> N-LINKED

<400> 50

```
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Asn Pro Ser
        35
```

<210> 51
<211> 39
<212> PRT
<213> Artificial Sequence

<220>
<221> AMIDATION
<222> 39
<223> Modified Exendin-4

<220>
<221> CARBOHYD
<222> 38
<223> N-LINKED

<400> 51

```
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Asp Ser
        35
```

<210> 52
<211> 39
<212> PRT
<213> Artificial Sequence

<220>
<221> AMIDATION
<222> 39
<223> Modified Exendin-4

<220>
<221> CARBOHYD
<222> 39

<223> N-LINKED

<400> 52

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Ser Gly Ala Pro Pro Pro Asp
35

<210> 53
<211> 40
<212> PRT
<213> Artificial Sequence

<220>
<221> AMIDATION
<222> 40
<223> Modified Exendin-4

<220>
<221> CARBOHYD
<222> 40
<223> N-LINKED

<400> 53

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Ser Gly Ala Pro Pro Pro Ser Asn
35 40

<210> 54
<211> 40
<212> PRT
<213> Artificial Sequence

<220>
<221> AMIDATION
<222> 40
<223> Modified Exendin-4

<220>
<221> CARBOHYD
<222> 28
<223> N-LINKED

<220>
<221> CARBOHYD
<222> 40
<223> N-LINKED

<400> 54

His Gly Glu Gly Thr Phe Thr Ser Asp Leu Ser Lys Gln Met Glu Glu
1 5 10 15

Glu Ala Val Arg Leu Phe Ile Glu Trp Leu Lys Asn Gly Gly Pro Ser
20 25 30

Ser Gly Ala Pro Pro Pro Ser Asn
35 40

<210> 55
<211> 40
<212> PRT
<213> Artificial sequence

<220>
<221> AMIDATION
<222> 40
<223> Modified Exendin-4

<220>
<221> CARBOHYD
<222> 17
<223> N-LINKED

<220>
<221> CARBOHYD
<222> 21
<223> N-LINKED

<220>
<221> CARBOHYD
<222> 28
<223> N-LINKED

<220>
<221> CARBOHYD
<222> 40
<223> N-LINKED

<400> 55

His Gly Glu Gly Thr Phe Thr Ser Asp Leu Ser Lys Gln Met Glu Glu
1 5 10 15

Asn Ala Val Arg Asn Phe Ile Glu Trp Leu Lys Asn Gly Gly Pro Ser

20 25

Ser Gly Ala Pro Pro Pro Ser Asn
35 40

<210> 56
<211> 28
<212> PRT
<213> Artificial Sequence

<220>
<221> AMIDATION
<222> 28
<223> Modified Exendin-4

<400> 56

```
His Gly Glu Gly Thr Phe Thr Ser Asp Leu Ser Lys Gln Met Glu Glu
1               5                   10                  15

Asn Ala Val Arg Asn Phe Ile Glu Trp Leu Lys Asn
            20                  25
```

<210> 57
<211> 39
<212> PRT
<213> Artificial sequence

<220>
<221> AMIDATION
<222> 39
<223> Modified Exendin-4

<220>
<221> CARBOHYD
<222> 28
<223> N-LINKED

<400> 57

```
His Gly Glu Gly Thr Phe Thr Ser Asp Leu Ser Lys Gln Met Glu Glu
1               5                   10                  15

Asn Ala Val Arg Asn Phe Ile Glu Trp Leu Lys Cys Gly Gly Pro Ser
            20                  25                  30

Ser Gly Ala Pro Pro Pro Ser
        35
```

<210> 58
<211> 40
<212> PRT
<213> Artificial Sequence

<220>
<221> AMIDATION
<222> 40
<223> Modified Exendin-4

<220>
<221> CARBOHYD
<222> 28
<223> N-LINKED

<220>
<221> CARBOHYD
<222> 40
<223> N-LINKED

<400> 58

His Gly Glu Gly Thr Phe Thr Ser Asp Leu Ser Lys Gln Met Glu Glu
1               5                   10                  15

Asn Ala Val Arg Asn Phe Ile Glu Trp Leu Lys Cys Gly Gly Pro Ser
            20                  25                  30

Ser Gly Ala Pro Pro Pro Ser Cys
        35                  40

<210> 59
<211> 40
<212> PRT
<213> Artificial sequence

<220>
<221> AMIDATION
<222> 40
<223> Modified Exendin-4

<220>
<221> CARBOHYD
<222> 21
<223> N-LINKED

<220>
<221> CARBOHYD
<222> 28
<223> N-LINKED

<220>
<221> CARBOHYD
<222> 35
<223> N-LINKED

<220>
<221> CARBOHYD
<222> 40
<223> N-LINKED

<400> 59

His Gly Glu Gly Thr Phe Thr Ser Asp Leu Ser Lys Gln Met Glu Glu
1               5                   10                  15

Asn Ala Val Arg Cys Phe Ile Glu Trp Leu Lys Cys Gly Gly Pro Ser
            20                  25                  30

Ser Gly Cys Pro Pro Pro Ser Cys
        35                  40